# EUROPEAN PATENT SPECIFICATION

(11) **EP 2 125 025 B1**
(45) Date of publication and mention of the grant of the patent: **14.11.2012**
(21) Application number: 08728317.2
(22) Date of filing: 25.01.2008
(51) Int. Cl.: A61K 47/48, A61K 49/08, A61K 49/00, A61K 49/14, A61P 35/00

(54) **MULTI-FUNCTIONAL DRUG CARRIERS**
MULTIFUNKTIONALE MEDIKAMENTENTRÄGER
SUPPORTS DE MÉDICAMENT MULTIFONCTIONNELS

(30) Priority: 29.01.2007 US 887011 P; 01.05.2007 US 915364 P
(43) Date of publication of application: 02.12.2009
(73) Proprietor: Nitto Denko Corporation, Ibaraki Osaka 567-8680 (JP)
(72) Inventor: YU, Lei, Carlsbad, CA 92008 (US); ZHAO, Gang, Vista, CA 92081 (US); VAN, Sang, San Diego, CA 92111 (US); DAS, Sanjib, Kumar, Oceanside, CA 92054 (US); CHEN, Fu, Carlsbad, CA 92008 (US); JIN, Yi, Carlsbad, CA 92008 (US); FU, Xiaoli, Vista, CA 92081 (US)
(74) Representative: Miles, John Stephen
(86) International application number: PCT/US2008/052094
(87) International publication number: WO 2008/094834

(56) References cited:
- EP-A- 1 580 216
- WO-A-02/087497
- WO-A-2005/079861
- WO-A-2006/060797
- WO-A-2007/067417
- WO-A-2008/141110

## Description

### BACKGROUND OF THE INVENTION

### Field of the Invention

This invention relates generally to biocompatible water-soluble polymers with pendant functional groups and methods for making them, and particularly to polyglutamate amino acid conjugates according to the claims useful for a variety of drug, biomolecule and imaging agent delivery applications.

### Description of the Related Art

A variety of systems have been used for the delivery of drugs, biomolecules, and imaging agents. For example, such systems include capsules, liposomes, microparticles, nanoparticles, and polymers.

A variety of polyester-based biodegradable systems have been characterized and studied. Polylactic acid (PLA), polyglycolic acid and their copolymers polylactic-co-glycolic acid (PLGA) are some of the most well-characterized biomaterials with regard to design and performance for drug-delivery applications. See Uhrich, K.E.; Cannizzaro, S.M.; Langer, R.S. and Shakeshelf, K.M. "Polymeric Systems for Controlled Drug Release," Chem. Rev. 1999, 99, 3181-3198 and Panyam J, Labhasetwar V. "Biodegradable nanoparticles for drug and gene delivery to cells and tissue," Adv. Drug. Deliv. Rev. 2003, 55, 329-47. Also, 2-hydroxypropyl methacrylate (HPMA) has been widely used to create a polymer for drug-delivery applications. Biodegradable systems based on polyorthoesters have also been investigated. See Heller, J.; Barr, J.; Ng, S.Y.; Abdellauoi, K.S. and Gurny, R. "Poly(ortho esters): synthesis, characterization, properties and uses." Adv. Drug Del. Rev. 2002, 54, 1015-1039. Polyanhydride systems have also been investigated. Such polyanhydrides are typically biocompatible and may degrade in vivo into relatively non-toxic compounds that are eliminated from the body as metabolites. See Kumar, N.; Langer, R.S. and Domb, A.J. "Polyanhydrides: an overview," Adv. Drug Del. Rev.. 2002, 54, 889-91.

Amino acid-based polymers have also been considered as a potential source of new biomaterials. Poly-amino acids having good biocompatibility have been investigated to deliver low molecular-weight compounds. A relatively small number of polyglutamic acids and copolymers have been identified as candidate materials for drug delivery. See Bourke, S.L. and Kohn, J. "Polymers derived from the amino acid L-tyrosine: polycarbonates, polyarylates and copolymers with poly(ethylene glycol)." Adv. Drug Del. Rev., 2003, 55, 447- 466.

Administered hydrophobic anticancer drugs and therapeutic proteins and polypeptides often suffer from poor bio-availability. Such poor bio-availability may be due to incompatibility of bi-phasic solutions of hydrophobic drugs and aqueous solutions and/or rapid removal of these molecules from blood circulation by enzymatic degradation. One technique for increasing the efficacy of administered proteins and other small molecule agents entails conjugating the administered agent with a polymer, such as a polyethylene glycol ("PEG") molecule, that can provide protection from enzymatic degradation in vivo. Such "PEGylation" often improves the circulation time and, hence, bio-availability of an administered agent.

PEG has shortcomings in certain respects, however. For example, because PEG is a linear polymer, the steric protection afforded by PEG is limited, as compared to branched polymers. Another shortcoming of PEG is that it is generally amenable to derivatization at its two terminals. This limits the number of other functional molecules (e.g. those helpful for protein or drug delivery to specific tissues) that can be conjugated to PEG.

Polyglutamic acid (PGA) is another polymer of choice for solubilizing hydrophobic anticancer drugs. WO2005/079861 discloses conjugates of polyglutamic acid to paclitaxel and PEG. Many anti-cancer drugs conjugated to PGA have been reported. See Chun Li. "Poly(L-glutamic acid)-anticancer drug conjugates." Adv. Drug Del. Rev., 2002, 54, 695-713. However, none are currently FDA-approved.

Paclitaxel, extracted from the bark of the Pacific Yew tree, is a FDA-approved drug for the treatment of ovarian cancer and breast cancer. Wani et al. "Plant antitumor agents. VI. The isolation and structure of taxol, a novel antileukemic and antitumor agent from Taxus brevifolia," J. Am. Chem. Soc. 1971, 93, 2325-7. However, like other anti-cancer drugs, pacilitaxel suffers from poor bio-availability due to its hydrophobicity and insolubility in aqueous solution. One way to solubilize pacilitaxel is to formulate it in a mixture of Cremophor-EL and dehydrated ethanol (1:1, v/v). Sparreboom et al. "Cremophor EL-mediated Alteration of Paclitaxel Distribution in . Human Blood: Clinical Pharmacokinetic Implications," Cancer Research, 1999, 59, 1454-1457. This formulation is currently commercialized as Taxol^{®} (Bristol-Myers Squibb). Another method of solubilizing paclitaxel is by emulsification using high-shear homogenization. Constantinides et al. "Formulation Development and Antitumor Activity of a Filter-Sterilizable Emulsion of Paclitaxel," Pharmaceutical Research 2000, 17, 175-182. Recently, polymer-paclitaxel conjugates have been advanced in several clinical trials. Ruth Duncan "The Dawning era of polymer therapeutics," Nature Reviews Drug Discovery 2003, 2, 347-360. More recently, paclitaxel has been formulated into nano-particles with human albumin protein and has been used in clinical studies. Damascelli et al. "Intraarterial chemotherapy with polyoxyethylated castor oil free paclitaxel, incorporated in albumin nanoparticles (ABI-007): Phase II study of patients with squamous cell carcinoma of the head and neck and anal canal: preliminary evidence of clinical activity." Cancer, 2001, 92, 2592-602, and Ibrahim et al. "Phase I and pharmacokinetic study of ABI-007, a Cremophor-free, protein-stabilized, nanoparticle formulation of paclitaxel," Clin. Cancer Res. 2002, 8, 1038-44. This formulation is currently commercialized as Abraxane^{®} (American Pharmaceutical Partners, Inc.).

Magnetic resonance imaging (MRI) is an important tool in diagnosis and staging of disease because it is non-invasive and non-irradiating. See Bulte et al. "Magnetic resonance microscopy and histology of the CNS," Trends in Biotechnology, 2002, 20, S24-S28). Although images of tissues can be obtained, MRI with contrast agents significantly improves its resolution. However, paramagnetic metal ions suitable for MRI contrast agents are often toxic. One of the methods to reduce toxicity is to chelate these metal ions with polydentate molecules such as diethylenetriamine pentaacetate molecules (DTPA). Gd-DTPA was approved by FDA in 1988 for clinical uses, and it is currently commercialized as Magnevist®. Other Gd-chelates were approved by FDA and commercialized, and many others are under development. See Caravan et al. "Gadolinium(III) Chelates as MRI Contrast Agents: Structure, Dynamics, and Applications," Chem. Rev. 1999, 99, 2293-2352.

However, Gd-DTPA is not ideal for targeting tumor tissues because it lacks specificity. When Gd-DTPA is administered via IV injection, it spontaneously and rapidly diffuses into extravascular space of the tissues. Thus, large amounts of contrast agents are usually required to produce reasonable contrast images. In addition, it is quickly eliminated via kidney filtration. To avoid the diffusion and the filtration, macromolecular MRI contrast agents have been developed. See Caravan et al. "Gadolinium(III) Chelates as MRI Contrast Agents: Structure, Dynamics, and Applications," Chem. Rev. 1999, 99, 2293-2352. These macromolecular-MRI contrast agents include protein-MRI chelates, polysaccharide-MRI chelates, and polymer-MRI chelates. See Lauffer et al. "Preparation and Water Relaxation Properties of Proteins Labeled with Paramagnetic Metal Chelates," Magn. Reson. Imaging 1985, 3, 11-16; Sirlin et al. "Gadolinium-DTPA-Dextran: A Macromolecular MR Blood Pool Contrast Agent," Acad. Radiol. 2004, 11, 1361-1369; Lu et al. "Poly(L-glutamic acid) Gd(III)-DOTA Conjugate with a Degradable Spacer for Magnetic Resonance Imaging," Bioconjugate Chem. 2003, 14, 715-719; and Wen et al. "Synthesis and Characterization of Poly(L-glutamic acid) Gadolinium Chelate: A New Biodegradable MRI Contrast Agent," Bioconjugate Chem. 2004, 15, 1408-1415.

Recently, tissue-specific MRI contrast agents have been developed. See Weinmann et al. "Tissue-specific MR contrast agents." Eur. J. Radiol. 2003, 46, 33-44. However, tumor-specific MRI contrast agents have not been reported in clinical applications. Nano-size particles have been reported to target tumor-tissues via an enhanced permeation and retention (EPR) effect. See Brannon-Peppas et al. "Nanoparticle and targeted systems for cancer therapy." ADDR, 2004, 56, 1649-1659).

### SUMMARY OF THE INVENTION

Relatively hydrophobic imaging agents and drugs (such as certain hydrophobic anti-cancer drugs, therapeutic proteins and polypeptides) often suffer from poor bioavailability. It is believed that this problem is due at least in part to the poor solubility of these imaging agents and drugs in aqueous systems. Certain enzymatically degradable drugs also suffer from poor bioavailability because they are degraded relatively rapidly in the circulatory system, resulting in rapid elimination from the body.

The inventors have discovered a series of novel polyglutamate-amino acids that are capable of conjugating to a number of agents, such as imaging agents, targeting agents and/or drugs. In certain embodiments, the polymers and the resulting conjugates preferentially accumulate in certain tissues (e.g., tumor tissues) and/or certain receptors, and thus are useful for delivering drugs (e.g., anticancer drugs) and/or imaging agents to specific parts of the body (e.g., tumors). In certain embodiments, the polymers and the resulting polymer conjugates form nanoparticles that effectively solubilize the imaging agent, targeting agent, magnetic resonance imaging agent, and/or drug in aqueous systems by dispersing it at a molecular level, thereby increasing functionality and/or bioavailability.

An embodiment provides a polymer conjugate comprising a recurring unit of the formula (I) and a recurring unit of the formula (II) as set forth herein, wherein: each n can be independently 1 or 2; each A¹ and A² can be independently oxygen or NR⁵, wherein R⁵ can be hydrogen or C₁₋₄ alkyl; and each R¹, R², R³ and R⁴ can be independently selected from the group consisting of hydrogen, a C₁₋₁₀ alkyl group, a C₆₋₂₀ aryl groups, ammonium, an alkali metal, a polydentate ligand, a polydentate ligand precursor with protected oxygen atoms, and a compound that comprises an agent, wherein each agent can be independently selected from the group consisting of a drug, a targeting agent, an optical imaging agent, a magnetic resonance imaging agent, and a stabilizing agent; wherein the drug is an anticancer drug; wherein the stabilizing agent is polyethylene glycol; and wherein the targeting agent is selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF) and an antibody; or wherein the targeting agent interacts with a receptor selected from the group consisting of αᵥ ,β₃ -integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor; provided that at least one of R¹ and R² is a compound that comprises an anticancer drug; and at least one of R³ and R⁴ is a polydentate ligand, a polydentate ligand precursor with protected oxygen atoms or a compound that comprises an agent selected from the group consisting of a targeting agent, an optical imaging agent, a magnetic resonance imaging agent, and polyethylene glycol.

Another embodiment provides a method of making the polymer conjugate described above, comprising dissolving or partially dissolving a polymeric reactant comprising a recurring unit of formula (V), as described herein, in a solvent to form a dissolved or partially dissolved polymeric reactant; wherein: each n can be independently 1 or 2; each A⁴ can be oxygen; and R¹¹ and R¹² can be each independently selected from the group consisting of hydrogen, ammonium, and an alkali metal; and reacting the dissolved or partially dissolved polymeric reactant with a second reactant and a third reactant, wherein the second reactant comprises the drug; and wherein the third reactant comprises the polydentate ligand, the polydentate ligand precursor with protected oxygen atoms or the compound that comprises the agent.

Another embodiment provides a pharmaceutical composition comprising the polymer conjugate described herein, and further comprising at least one selected from a pharmaceutically acceptable excipient, a carrier, and a diluent.

Another embodiment provides the polymer conjugate described herein for use in treating or ameliorating a disease or condition in a mammal in need thereof.

Another embodiment provides the polymer conjugate described herein for use diagnosing a disease or condition in a mammal.

Another embodiments provides a method of imaging a portion of tissue comprising contacting a portion of tissue with an effective amount of the polymer conjugate described herein.

These and other embodiments are described in greater detail below.

### BRIEF DESCRIPTION OF THE DRAWINGS

Figure ·1 illustrates a reaction scheme for the preparation of poly-(γ-L-glutamyl-glutamine)-doxorubicin, PGGA-Dox.

Figure 2 illustrates a reaction scheme for the preparation of (cyclic(fKRGD))-poly-(γ-L-glutamyl-glutamine)-doxorubicin, RGD-PGGA-Dox.

Figure 3 illustrates a reaction scheme for the preparation of polyethyleneglycol-poly-(γ-L-glutamyl-glutamine)-doxorubicin, PEG-PGGA-Dox.

Figure 4 illustrates a reaction scheme for the preparation of polyethyleneglycol-poly-(γ-L-glutamyl-glutamine)- doxorubicin -(cyclic(fKRGD)), PEG-PGGA-Dox-RGD

Figure 5 illustrates a reaction scheme for the preparation of polyethyleneglycol-poly-(γ-L-glutamyl-glutamine)- doxorubicin -NHCH₂CH₂NHBoc, PEG-PGGA-Dox-NHCH₂CH₂NHBoc

Figure 6 illustrates a reaction scheme for the preparation of polyethyleneglycol-poly-(y-L-glutamyl-glutamine)- doxorubicin -(cyclic(fKRGD))-diethylenetriaminepentaacetic acid, PEG-PGGA-Dox-RGD-DTPA

Figure 7 illustrates a reaction scheme for the preparation of polyethyleneglycol-poly-(y-L-glutamyl-glutamine)- doxorubicin -(cyclic(fKRGD))-diethylenetriaminepentaacetate[Gd(III)], PEG-PGGA-Dox-RGD-[(DTPA)Gd(III)]

### DETAILED DESCRIPTION OF THE PREFERRED EMBODIMENTS

Unless defined otherwise, all technical and scientific terms used herein have the same meaning as is commonly understood by one of ordinary skill in the art. In the event that there are a plurality of definitions for a term herein, those in this section prevail unless stated otherwise.

The term "ester" is used herein in its ordinary sense, and thus includes a chemical moiety with formula -(R)ₙ-COOR', where R and R' are independently selected from the group consisting of alkyl, cycloalkyl, aryl, heteroaryl (bonded through a ring carbon) and heteroalicyclic (bonded through a ring carbon), and where n is 0 or 1.

The term "amide" is used herein in its ordinary sense, and thus includes a chemical moiety with formula -(R)ₙ-C(O)NHR' or -(R)ₙ-NHC(O)R', where R and R' are independently selected from the group consisting of alkyl, cycloalkyl, aryl, heteroaryl (bonded through a ring carbon) and heteroalicyclic (bonded through a ring carbon), and where n is 0 or 1. An amide may be included in an amino acid or a peptide molecule attached to drug molecule as described herein, thereby forming a prodrug.

Any amine, hydroxy, or carboxyl side chain on the compounds disclosed herein can be esterified or amidified. The procedures and specific groups to be used to achieve this end are known to those of skill in the art and can readily be found in reference sources such as Greene and Wuts, Protective Groups in Organic Synthesis, 3rd Ed., John Wiley & Sons, New York, NY, 1999.

As used herein, "alkyl" refers to a straight or branched hydrocarbon chain that comprises a fully saturated (no double or triple bonds) hydrocarbon group. The alkyl group may have 1 to 20 carbon atoms (whenever it appears herein, a numerical range such as "1 to 20" refers to each integer in the given range; e.g., "1 to 20 carbon atoms" means that the alkyl group may consist of 1 carbon atom, 2 carbon atoms, 3 carbon atoms, *etc.,* up to and including 20 carbon atoms, although the present definition also covers the occurrence of the term "alkyl" where no numerical range is designated). The alkyl group may also be a medium size alkyl having 1 to 10 carbon atoms. The alkyl group could also be a lower alkyl having 1 to 5 carbon atoms. The alkyl group of the compounds may be designated as "C₁-C₄ alkyl" or similar designations. By way of example only, "C₁-C₄ alkyl" indicates that there are one to four carbon atoms in the alkyl chain, i.e., the alkyl chain is selected from the group consisting of methyl, ethyl, propyl, iso-propyl, n-butyl, iso-butyl, sec-butyl, and t-butyl. Typical alkyl groups include methyl, ethyl, propyl, isopropyl, butyl, isobutyl, tertiary butyl, pentyl, hexyl.

The alkyl group may be substituted or unsubstituted. When substituted, the substituent group(s) is(are) one or more group(s) individually and independently selected from alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxyl, alkoxy, aryloxy, acyl, ester, mercapto, alkylthio, arylthio, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl (e.g., mono-, di- and tri-haloalkyl), haloalkoxy (e.g., mono-, di- and tri-haloalkoxy), trihalomethanesulfonyl, trihalomethanesulfonamido, and amino, including mono- and di-substituted amino groups, and the protected derivatives thereof. Wherever a substituent is described as being "optionally substituted" that substitutent may be substituted with one of the above substituents.

As used herein, "aryl" refers to a carbocyclic (all carbon) monocyclic or multicyclic aromatic ring system that has a fully delocalized pi-electron system. Examples of aryl groups include benzene, naphthalene and azulene. An aryl group of this invention may be substituted or unsubstituted. When substituted, hydrogen atoms are replaced by substituent group(s) that is(are) one or more group(s) independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxy, alkoxy, aryloxy, acyl, ester, mercapto, cyano, halogen, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof, unless the substituent groups are otherwise indicated.

A "paramagnetic metal chelate" is a complex wherein a ligand is bound to a paramagnetic metal ion. Examples include 1,4,7,10-Tetraazacyclododecane-1,4,7,10-tetraacetic acid (DOTA)-Gd(III), DOTA-Yttrium-88, DOTA-Indium-111, diethylenetriaminepentaacetic acid (DTPA)-Gd(III), DTPA-yttrium-88, DTPA-Indium-111.

A "polydentate ligand" is a ligand that can bind itself through two or more points of attachment to a metal ion through, for example, coordinate covalent bonds. Examples of polydentate ligands include, diethylenetriaminepentaacetic acid (DTPA), tetraazacyclododecane-1,4,7,10-tetraacetic acid (DOTA), (1,2-ethanediyldinitrilo)tetraacetate (EDTA), ethylenediamine, 2,2'-bipyridine (bipy), 1,10-phenanthroline (phen). 1,2-bis(diphenylphosphino)ethane (DPPE), 2,4-pentanedione (acac), and ethanedioate (ox).

A "polydentate ligand precursor with protected oxygen atoms" is a polydentate ligand comprising oxygen atoms, such as the single-bonded oxygen atoms of carboxyl groups, that are protected with suitable protecting groups. Suitable protecting groups include, lower alkyls, benzyls, and silyl groups.

A "stabilizing agent" is a substituent that enhances bioavailability and/or prolongs the half-life of a carrier-drug conjugate in vivo by rendering it more resistant to hydrolytic enzymes and less immunogenic. The stabilizing agent is polyethylene glycol (PEG).

It is understood that, in any compound described herein having one or more chiral centers, if an absolute stereochemistry is not expressly indicated, then each center may independently be of R-configuration or S-configuration or a mixture thereof. Thus, the compounds provided herein may be enatiomerically pure or be stereoisomeric mixtures. In addition it is understood that, in any compound described herein having one or more double bond(s) generating geometrical isomers that can be defined as E or Z each double bond may independently be E or Z a mixture thereof. Likewise, all tautomeric forms are also intended to be included.

[0037] An embodiment provides a polymer conjugate comprising a recurring unit of the formula (I) and a recurring unit of the formula (II): 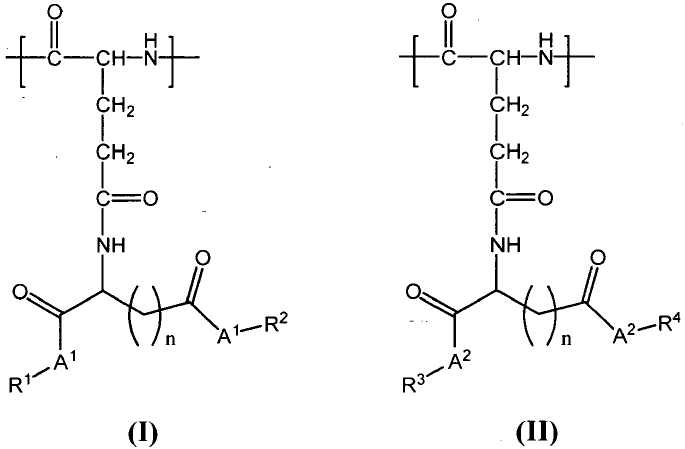 wherein: each n can be independently 1 or 2, each A¹ and A² can be independently oxygen or NR⁵, wherein R⁵ can be hydrogen or C₁₋₄ alkyl, and each R¹, R², R³ and R⁴ can be independently selected from the group consisting of hydrogen, a C₁₋₁₀ alkyl group, a C₆₋₂₀ aryl group, ammonium, an alkali metal, a polydentate ligand, a polydentate ligand precursor with protected oxygen atoms, and a compound that comprises an agent, wherein each agent can be independently selected from the group consisting of a drug, a targeting agent, an optical imaging agent, a magnetic resonance imaging agent, and a stabilizing agent; wherein the drug is an anticancer drug; wherein the stabilizing agent is polyethylene glycol; and wherein the targeting agent is selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF) and an antibody; or wherein the targeting agent interacts with a receptor selected from the group consisting of αᵥ β₃ -integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor; provided that at least one of R¹ and R² is a compound that comprises an anticancer drug, and at least one of R³ and R⁴ is a polydentate ligand, a polydentate ligand precursor with protected oxygen atoms or a compound that comprises an agent selected from the group consisting of a targeting agent, an optical imaging agent, a magnetic resonance imaging agent, and polyethylene glycol. Examples of alkali metal include lithium (Li), sodium (Na), potassium (K), rubidium (Rb) and cesium (Cs). In an embodiment, the alkali metal is sodium.

The agent(s) may comprise any number of active compounds. For instance, the agent may be selected from the group consisting of an anticancer drug, a targeting agent, an optical imaging agent, a magnetic resonance imaging agent, and polyethylene glycol. At least one of R¹ and R² is a compound that comprises an anticancer drug, and at least one of R³ and R⁴ is a compound that comprises an agent selected from the group consisting of a targeting agent, an optical imaging agent, a magnetic resonance imaging agent, and polyethylene glycol. In an embodiment, R¹ and R² are each independently selected from the group consisting of hydrogen, ammonium, and an alkali metal. In an embodiment, R³ and R⁴ are each independently selected from the group consisting of hydrogen, ammonium, and an alkali metal. In another embodiment, R⁵ is either a hydrogen atom or a C₁₋₄ alkyl group.

The amount of agent(s) present in the polymer conjugate can vary over a wide range. In an embodiment, the polymer conjugate comprises an amount of the agent(s) in the range of about 1 to about 50% (weight/weight) based on the mass ratio of the agent(s) to the polymer conjugate. In another embodiment, the polymer conjugate comprises an amount of the agent(s) in the range of about 5 to about 40% (weight/weight) based on the mass ratio of the agent(s) to the polymer conjugate. In another embodiment, the polymer conjugate comprises an amount of the agent(s) in the range of about 10 to about 30% (weight/weight) based on the mass ratio of the agent(s) to the polymer conjugate.

It has now been found that the amount of the agent(s) and the percentage amounts of the recurring units of the formula (I) and formula (II) may be selected to advantageously control the solubility of the resulting polymer conjugate. For example, in preferred embodiments, the amount of the agent(s) and the percentage amounts of the recurring units of the formula (I) and formula (II) are selected so that the polymer conjugate is soluble (or insoluble) at a particular pH and/or pH range of interest. In some embodiments, the molecular weight of the polymer is also selected to control solubility. Examples provided below illustrate control over solubility by appropriate selection of the amount of the agent, the percentage amounts of the recurring units of the formula (I) and formula (II), and molecular weight. Those skilled in the art, informed by the guidance provided herein, can use routine experimentation to identify suitable amounts of the agent(s) and percentage amounts of the recurring units of the formula (I) and formula (II) that result in a polymer conjugate with desired solubility characteristics. Such control over solubility may be advantageous, depending on the application. For example, embodiments of the polymer conjugates provided herein may be used to provide improved delivery of otherwise poorly soluble anticancer drugs to selected tissues, preferably reducing undesired side effects, and/or may reduce the frequency at which a subject needs to take the anticancer drug.

The amount of the agent(s) and the percentage amounts of the recurring units of the formula (I) and formula (II) are preferably selected to provide a polymer conjugate solubility that is greater than that of a comparable polyglutamic acid conjugate that comprises substantially the same amount of the same agent(s). In an embodiment, the polymer conjugate solubility is greater than that of a comparable polyglutamic acid conjugate. Solubility is measured by forming a polymer conjugate solution comprising at least 5 mg/mL of the polymer conjugate in 0.9 wt. % aqueous NaCl at about 22°C, and determining the optical clarity. Optical clarity may be determined turbidimetrically, e.g., by visual observation or by appropriate instrumental methods known to those skilled in the art. Comparison of the resulting solubility to a similarly formed polyglutamic acid conjugate solution shows improved solubility as evidenced by greater optical clarity over a broader range of pH values. Thus, a polymer conjugate solubility is greater than that of a comparable polyglutamic acid conjugate that comprises substantially the same amount of the agent when a tested polymer conjugate solution, comprising at least 5 mg/mL of the polymer conjugate in 0.9 wt. % aqueous NaCl at about 22°C, has greater optical clarity over a broader pH range than that of a comparable tested polyglutamic acid conjugate solution. Those skilled in the art will understand that a "comparable" polyglutamic acid conjugate is a control material in which the polymeric portion of the conjugate has a molecular weight that is approximately the same as that of the subject polymer conjugate (comprising a recurring unit of the formula (I) and a recurring unit of the formula (II)) to which it is being compared.

The polymer conjugate can contain one or more chiral carbon atoms. The chiral carbon (which may be indicated by an asterisk *) can have the *rectus* (right handed) or the *sinister* (left handed) configuration, and thus the recurring unit may be racemic, enantiomeric or enantiomerically enriched. The symbols "n" and "*" (designating a chiral carbon), as used elsewhere herein, have the same meaning as specified above, unless otherwise stated.

Polymers comprising a recurring unit of the formula (I) and a recurring unit of the formula (II) are copolymers comprising two or more different recurring units of the formula (I) and the formula (II). Further, polymers comprising a recurring unit of the formula (I) and a recurring unit of the formula (II) may be copolymers that comprise other recurring units that are not of the formula (I) and not of the formula (II). The number of recurring units of the formula (I) and recurring units of formula (II) in the polymer is not limited, but is preferably in the range of from about 50 to about 5,000, and more preferably from about 100 to about 2,000.

A broad variety of other recurring units may be included in the polymer conjugate with the recurring unit of formula (I) and the recurring unit of formula (II). In some embodiments, the polymer conjugate further comprises a recurring unit of formula (III) 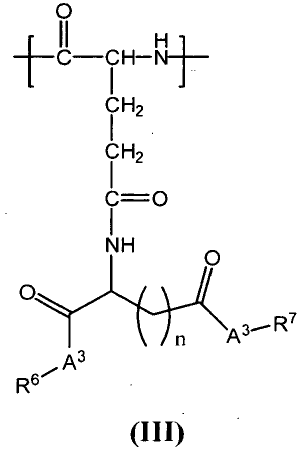 wherein each A³ is oxygen; and R⁶ and R⁷ are each independently selected from the group consisting of hydrogen, ammonium, and an alkali metal. In certain embodiments, the recurring unit of formula (III) is present in a sufficient amount to modulate solubility (e.g., increase solubility).

In an embodiment, the polymer conjugate further comprises a recurring unit of the formula (IV): 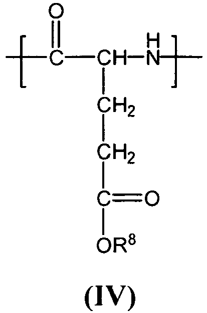 wherein the R⁸ group is hydrogen, ammonium, or an alkali metal. When the R⁸ group is hydrogen, then the recurring unit of the formula (IV) is a recurring unit of glutamic acid.

The compound that comprises the agent may be conjugated to the polymer in many different ways. In some embodiments, the compound that comprises the agent can be directly attached to the polymer. In one embodiment, the agent can be directly attached to the polymer through an oxygen, a sulfur, a nitrogen and/or carbon atom of the agent. In an embodiment, the agent can be directly attached to a recurring unit of Formula (I) or (II). In other embodiments, the compound that comprises the agent further comprises a linker group. A linker group is a group that attaches the agent (or the compound that comprises the agent) to the polymer. In an embodiment, the agent can be attached to a recurring unit of Formula (I) or (II) through a linker group. The linker group may be relatively small. For instance, the linker group may comprise an amine, an amide, an ether, an ester, a hydroxyl group, a carbonyl group, or a thiol group. Alternatively, the linker group may be relatively large. For instance, the linker group may comprise an alkyl group, an alkoxy group, an aryl group, an aryl(C₁₋₆ alkyl) group, a heteroaryl group, or a heteroaryl (C₁₋₆ alkyl) group. In one embodiment, the linker can be ―NH(CH₂)₁₋₄-NH-. In another embodiment, the linker can be -(CH₂)₁₋₄-aryl-NH-. The linker group can be attached to the agent at any suitable position. For example, the linker group can be attached in place of a hydrogen at a carbon of the agent. The linker group can be added to the agent using methods known to those skilled in the art.

The agent may comprise any type of active compound. In an embodiment, the agent may be an optical imaging agent. In a preferred embodiment, the optical imaging agent is one or more selected from the group consisting of an acridine dye, a coumarine dye, a rhodamine dye, a xanthene dye, a cyanine dye, and a pyrene dye. For instance, specific optical imaging agents may include Texas Red, Alexa Fluor® dye, BODIPY® dye, Fluorescein, Oregon Green® dye, and Rhodamine Gree™ dye, which are commercially available or readily prepared by methods known to those skilled in the art.

In another embodiment, the agent comprises an anticancer drug. In an embodiment, the anticancer drug may be selected from the group consisting of a taxane, camptotheca, and anthracycline. When the agent comprises a taxane, it is preferable that the taxane is paclitaxel or docetaxel. Paclitaxel may be conjugated to the recurring unit of formula (I) or the recurring unit of formula (II) at the oxygen atom via the C2'-carbon of the paclitaxel. Alternatively or in addition, paclitaxel may be conjugated to the recurring unit of formula (I) or the recurring unit of formula (II) at the oxygen atom via the C7-carbon of the paclitaxel. When the anticancer drug is a camptotheca, it is preferably camptothecin. In an embodiment when the anticancer drug is anthracycline, it can be doxorubicin.

In another embodiment, the agent may be a targeting agent. The targeting agent is one or more selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF), and an antibody. In another preferred embodiment, the targeting agent can interact with a receptor selected from the group consisting of α_{v,}β₃-integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor. In an embodiment, the arginine-glycine-aspartate (RGD) peptide is cyclic(fKRGD).

In another embodiment, the agent comprises a magnetic resonance imaging agent. In an embodiment, the magnetic resonance imaging agent comprises a paramagnetic metal compound. For example, the magnetic resonance imaging agent may comprise a Gd(III) compound. In an embodiment, the Gd(III) compound can be selected from the group consisting of:

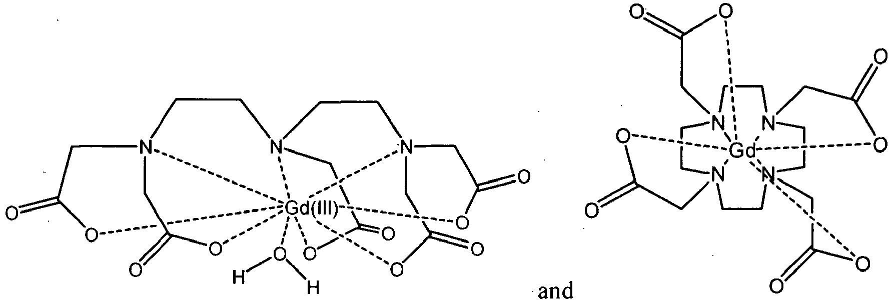

In another embodiment, the agent comprises the stabilizing agent polyethylene glycol.

In another embodiment, the polymer conjugate comprises a polydentate ligand. In an embodiment, the polydentate ligand may be capable of reaction with a paramagnetic metal to form a magnetic resonance imaging agent. The polydentate ligand may comprise several carboxylic acid and/or carboxylate groups. In an embodiment, the polydentate ligand can be selected from the group consisting of: 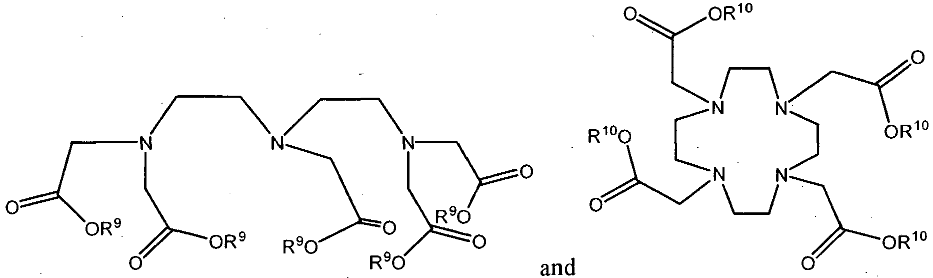 wherein each R⁹ and R¹⁰ are independently hydrogen, ammonium, or an alkali metal.

In another embodiment, the polymer conjugate comprises a polydentate ligand precursor. In such an embodiment, the oxygen atoms of the polydentate ligand are protected by a suitable protecting group. Suitable protecting groups include lower alkyls, benzyls, and silyl groups. One example of a polydentate ligand precursor having protecting groups is provided as follows:

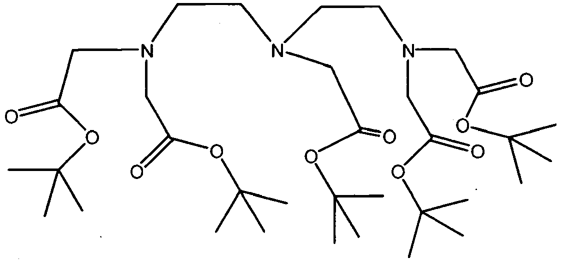

The percentage of recurring units of formula (I) in the polymer conjugate may vary over a wide range. In an embodiment, the polymer conjugate may comprise about 1 mole % to about 99 mole % of the recurring unit of formula (I), based on the total moles of recurring units of formulae (I) and (II) in the polymer conjugate. In another embodiment, the polymer conjugate may comprise about 1 mole % to about 50 mole % of the recurring unit of formula (I) based on the total moles of recurring units of formulae (I) and (II) in the polymer conjugate. In another embodiment, the polymer conjugate may comprise about 1 mole % to about 30 mole % of the recurring unit of formula (I) based on the total moles of recurring units of formulae (I) and (II) in the polymer conjugate. In another embodiment, the polymer conjugate may comprise about 1 mole % to about 20 mole % of the recurring unit of formula (I) based on the total moles of recurring units of formulae (I) and (II) in the polymer conjugate. In another embodiment, the polymer conjugate may comprise about 1 mole % to about 10 mole % of the recurring unit of formula (I) based on the total moles of recurring units of formulae (I) and (II) in the polymer conjugate.

The percentage of recurring units of formula (I) in the polymer conjugate, based on the total number of recurring units, may vary over a wide range. In an embodiment, the polymer conjugate may comprise about 1 mole % to about 99 mole % of the recurring unit of formula (I), based on the total moles of recurring units in the polymer conjugate. In another embodiment, the polymer conjugate may comprise about 1 mole % to about 50 mole % of the recurring unit of formula (I) based on the total moles of recurring units in the polymer conjugate. In another embodiment, the polymer conjugate may comprise about 1 mole % to about 30 mole % of the recurring unit of formula (I) based on the total moles of recurring units in the polymer conjugate. In another embodiment, the polymer conjugate may comprise about 1 mole % to about 20 mole % of the recurring unit of formula (I) based on the total moles of recurring units in the polymer conjugate. In another embodiment, the polymer conjugate may comprise about 1 mole % to about 10 mole % of the recurring unit of formula (I) based on the total moles of recurring units in the polymer conjugate.

In addition to recurring units of the formulae (I) and (II), the polymer conjugate may comprise a variety of other recurring units. For example, in an embodiment, the polymer conjugate comprises recurring units of the formula (III). The percentage of recurring units of formula (I), based on the total number of recurring units in a polymer conjugate comprising recurring units of formulae (I), (II), and (III), may vary over a wide range. In an embodiment, the polymer conjugate may comprise about 1 mole % to about 99 mole % of the recurring unit of formula (I) based on the total moles of recurring units of formulae (I), (II) and (III) in the polymer conjugate. In another embodiment, the polymer conjugate may comprise about 1 mole % to about 50 mole % of the recurring unit of formula (I) based on the total moles of recurring units of formulae (I), (II) and (III) in the polymer conjugate. In another embodiment, the polymer conjugate may comprise about 1 mole % to about 30 mole % of the recurring unit of formula (I) based on the total moles of recurring units of formulae (I), (II) and (III) in the polymer conjugate. In another embodiment, the polymer conjugate may comprise about 1 mole % to about 20 mole % of the recurring unit of formula (I) based on the total moles of recurring units of formulae (I), (II) and (III) in the polymer conjugate. In another embodiment, the polymer conjugate may comprise about 1 mole % to about 10 mole % of the recurring unit of formula (I) based on the total moles of recurring units of formulae (I), (II) and (III) in the polymer conjugate.

Similarly, in an embodiment, the polymer conjugate comprises recurring units of the formula (IV). The percentage of recurring units of formula (I), based on the total number of recurring units in a polymer conjugate comprising recurring units of formulae (I), (II), and (IV), may vary over a wide range. In an embodiment, the polymer conjugate may comprise about 1 mole % to about 99 mole % of the recurring unit of formula (I) based on the total moles of recurring units of formulae (I), (II) and (IV) in the polymer conjugate. In another embodiment, the polymer conjugate may comprise about 1 mole % to about 50 mole % of the recurring unit of formula (I) based on the total moles of recurring units of formulae (I), (II) and (IV) in the polymer conjugate. In another embodiment, the polymer conjugate may comprise about 1 mole % to about 30 mole % of the recurring unit of formula (I) based on the total moles of recurring units of formulae (I), (II) and (IV) in the polymer conjugate. In another embodiment, the polymer conjugate may comprise about 1 mole % to about 20 mole % of the recurring unit of formula (I) based on the total moles of recurring units of formulae (I), (II) and (IV) in the polymer conjugate. In another embodiment, the polymer conjugate may comprise about 1 mole % to about 10 mole % of the recurring unit of formula (I) based on the total moles of recurring units of formulae (I), (II) and (IV) in the polymer conjugate.

In another embodiment, the percentage of recurring units of formula (I), based on the total number of recurring units in a polymer conjugate comprising recurring units of formulae (I), (II), (III).and (IV), may vary over a wide range. In an embodiment, the polymer conjugate may comprise about 1 mole % to about 99 mole % of the recurring unit of formula (I) based on the total moles of recurring units of formulae (I), (II), (III) and (IV) in the polymer conjugate. In another embodiment, the polymer conjugate may comprise about 1 mole % to about 50 mole % of the recurring unit of formula (I) based on the total moles of recurring units of formulae (I), (II), (III) and (IV) in the polymer conjugate. In another embodiment, the polymer conjugate may comprise about 1 mole % to about 30 mole % of the recurring unit of formula (I) based on the total moles of recurring units of formulae (I), (II), (III) and (IV) in the polymer conjugate. In another embodiment, the polymer conjugate may comprise-about 1 mole % to about 20 mole % of the recurring unit of formula (I) based on the total moles of recurring units of formulae (I), (II), (III) and (IV) in the polymer conjugate. In another embodiment, the polymer conjugate may comprise about 1 mole % to about 10 mole % of the recurring unit of formula (I) based on the total moles of recurring units of formulae (I), (II), (III) and (IV) in the polymer conjugate.

The percentage of recurring units of formula (II) in the polymer conjugate may vary over a wide range. In an embodiment, the polymer conjugate may comprise about 1 mole % to about 99 mole % of the recurring unit of formula (II), based on the total moles of recurring units of formulae (I) and (II) in the polymer conjugate. In another embodiment, the polymer conjugate may comprise about 1 mole % to about 50 mole % of the recurring unit of formula (II) based on the total moles of recurring units of formulae (I) and (II) in the polymer conjugate. In another embodiment, the polymer conjugate may comprise about 1 mole % to about 30 mole % of the recurring unit of formula (II) based on the total moles of recurring units of formulae (I) and-(II) in the polymer conjugate: In another embodiment, the polymer conjugate may comprise about 1 mole % to about 20 mole % of the recurring, unit of formula (II) based on the total moles of recurring units of formulae (I) and (II) in the polymer conjugate. In another embodiment, the polymer conjugate may comprise about 1 mole % to about 10 mole % of the recurring unit of formula (II) based on the total moles of recurring units of formulae (I) and (II) in the polymer conjugate.

The percentage of recurring units of formula (II) in the polymer conjugate, based on the total number of recurring units, may vary over a wide range. In an embodiment, the polymer conjugate may comprise about 1 mole % to about 99 mole % of the recurring unit of formula (II), based on the total moles of recurring units in the polymer conjugate. In another embodiment, the polymer conjugate may comprise about 1 mole % to about 50 mole % of the recurring unit of formula (II) based on the total moles of recurring units in the polymer conjugate. In another embodiment, the polymer conjugate may comprise about 1 mole % to about 30 mole % of the recurring unit of formula (II) based on the total moles of recurring units in the polymer conjugate. In another embodiment, the polymer conjugate may comprise about 1 mole % to about 20 mole % of the recurring unit of formula (II) based on the total moles of recurring units in the polymer conjugate. In another embodiment, the polymer conjugate may comprise about 1 mole % to about 10 mole % of the recurring unit of formula (II) based on the total moles of recurring units in the polymer conjugate.

In addition to recurring units of the formulae (I) and (II), the polymer conjugate may comprise a variety of other recurring units. For example, in an embodiment, the polymer conjugate comprises recurring units of the formula (III) and/or (IV). The percentage of recurring units of formula (II) may vary over a wide range. In an embodiment, the polymer conjugate may comprise about 1 mole % to about 99 mole % of the recurring unit of formula (II) based on the total moles of recurring units of formulae (I), (II) and (III) in the polymer conjugate. In another embodiment, the polymer conjugate may comprise about 1 mole % to about 50 mole % of the recurring unit of formula (II) based on the total moles of recurring units of formulae (I), (II) and (III) in the polymer conjugate. In another embodiment, the polymer conjugate may comprise about 1 mole % to about 30 mole % of the recurring unit of formula (II) based on the total moles.of recurring units of formulae (I), (II) and (III) in the polymer conjugate. In another embodiment, the polymer conjugate may comprise about 1 mole % to about 20 mole % of the recurring unit of formula (II) based on the total moles of recurring units of formulae (I), (II) and (III) in the polymer conjugate. In another embodiment, the polymer conjugate may comprise about 1 mole % to about 10 mole % of the recurring unit of formula (II) based on the total moles of recurring units of formulae (I), (II) and (III) in the polymer conjugate.

Similarly, in an embodiment, the polymer conjugate comprises recurring units of the formula (IV). In an embodiment, the polymer conjugate may comprise about 1 mole % to about 99 mole % of the recurring unit of formula (II) based on the total moles of recurring units of formulae (I), (II) and (IV) in the polymer conjugate. In another embodiment, the polymer conjugate may comprise about 1 mole % to about 50 mole % of the recurring unit of formula (II) based on the total moles of recurring units of formulae (I), (II) and (IV) in the polymer conjugate. In another embodiment, the polymer conjugate may comprise about 1 mole % to about 30 mole % of the recurring unit of formula (II) based on the total moles of recurring units of formulae (I), (II) and (IV) in the polymer conjugate. In another embodiment, the polymer conjugate may comprise about 1 mole % to about 20 mole % of the recurring unit of formula (II) based on the total moles of recurring units of formulae (I), (II) and (IV) in the polymer conjugate. In another embodiment, the polymer conjugate may comprise about 1 mole % to about 10 mole % of the recurring unit of formula (II) based on the total moles of recurring units of formulae (I), (II) and (IV) in the polymer conjugate.

In another embodiment, the polymer conjugate may comprise about 1 mole % to about 99 mole % of the recurring unit of formula (II) based on the total moles of recurring units of formulae (I), (II), (III) and (IV) in the polymer conjugate. In another embodiment, the polymer conjugate may comprise about 1 mole % to about 50 mole % of the recurring unit of formula (II) based on the total moles of recurring units of formulae (I), (II), (III) and (IV) in the polymer conjugate. In another embodiment, the polymer conjugate may comprise about 1 mole % to about 30 mole % of the recurring unit of formula (II) based on the total moles of recurring units of formulae (I), (II), (III) and (IV) in the polymer conjugate. In another embodiment, the polymer conjugate may comprise about 1 mole % to about 20 mole % of the recurring unit of formula (II) based on the total moles of recurring units of formulae (I), (II), (III) and (IV) in the polymer conjugate. In another embodiment, the polymer conjugate may comprise about 1 mole % to about 10 mole % of the recurring unit of formula (II) based on the total moles of recurring units of formulae (I), (II), (III) and (IV) in the polymer conjugate.

The percentage of recurring units of formula (III) in the polymer conjugate may vary over a wide range. In an embodiment, the polymer conjugate may comprise about 1 mole % to about 99 mole % of the recurring unit of formula (III) based on the total moles of recurring units of formulae (I), (II) and (III) in the polymer conjugate. In another embodiment, the polymer conjugate may comprise about 1 mole % to about 50 mole % of the recurring unit of formula (III) based on the total moles of recurring units of formulae (I), (II) and (III) in the polymer conjugate. In another embodiment, the polymer conjugate may comprise about 1 mole % to about 30 mole % of the recurring unit of formula (III) based on the total moles of recurring units of formulae (I), (II) and (III) in the polymer conjugate. In another embodiment, the polymer conjugate may comprise about 1 mole % to about 20 mole % of the recurring unit of formula (III) based on the total moles of recurring units of formulae (I), (II) and (III) in the polymer conjugate. In another embodiment, the polymer conjugate may comprise about 1 mole % to about 10 mole % of the recurring unit of formula (III) based on the total moles of recurring units of formulae (I), (II) and (III) in the polymer conjugate.

In another embodiment, the polymer conjugate may comprise about 1 mole % to about 99 mole % of the recurring unit of formula (III) based on the total moles of recurring units of formulae (I), (II), (III) and (IV) in the polymer conjugate. In another embodiment, the polymer conjugate may comprise about 1 mole % to about 50 mole % of the recurring unit of formula (III) based on the total moles of recurring units of formulae (I), (II), (III) and (IV) in the polymer conjugate. In another embodiment, the polymer conjugate may comprise about 1 mole % to about 30 mole % of the recurring unit of formula (III) based on the total moles of recurring units of formulae (I), (II), (III) and (IV) in the polymer conjugate. In another embodiment, the polymer conjugate may comprise about 1 mole % to about 20 mole % of the recurring unit of formula (III) based on the total moles of recurring units of formulae (I), (II), (III) and (IV) in the polymer conjugate. In another embodiment, the polymer conjugate may comprise about 1 mole % to about 10 mole % of the recurring unit of formula (III) based on the total moles of recurring units of formulae (I), (II), (III) and (IV) in the polymer conjugate.

The percentage of recurring units of formula (IV) in the polymer conjugate may vary over a wide range. In an embodiment, the polymer conjugate may comprise about 1 mole % to about 99 mole % of the recurring unit of formula (IV) based on the total moles of recurring units of formulae (I), (II) and (IV) in the polymer conjugate. In another embodiment, the polymer conjugate may comprise about 1 mole % to about 50 mole % of the recurring unit of formula (IV) based on the total moles of recurring units of formulae (I), (II) and (IV) in the polymer conjugate. In another embodiment, the polymer conjugate may comprise about 1 mole % to about 30 mole % of the recurring unit of formula (IV) based on the total moles of recurring units of formulae (I), (II) and (IV) in the polymer conjugate. In another embodiment, the polymer conjugate may comprise about 1 mole % to about 20 mole % of the recurring unit of formula (IV) based on the total moles of recurring units of formulae (I), (II) and (IV). In another embodiment, the polymer conjugate may comprise about 1 mole % to about 10 mole % of the recurring unit of formula (IV) based on the total moles of recurring units of formulae (I), (II) and (IV) in the polymer conjugate.

In another embodiment, the polymer conjugate may comprise about 1 mole % to about 99 mole % of the recurring unit of formula (IV) based on the total moles of recurring units of formulae (I), (II), (III) and (IV) in the polymer conjugate. In another embodiment, the polymer conjugate may comprise about 1 mole % to about 50 mole % of the recurring unit of formula (IV) based on the total moles of recurring units of formulae (I), (II), (III) and (IV) in the polymer conjugate. In another embodiment, the polymer conjugate may comprise about 1 mole % to about 30 mole % of the recurring unit of formula (IV) based on the total moles of recurring units of formulae (I), (II), (III) and (IV) in the polymer conjugate. In another embodiment, the polymer conjugate may comprise about 1 mole % to about 20 mole % of the recurring unit of formula (IV) based on the total moles of recurring units of formulae (I), (II), (III) and (IV) in the polymer conjugate. In another embodiment, the polymer conjugate may comprise about 1 mole % to about 10 mole % of the recurring unit of formula-(IV) based on the total moles of recurring units of formulae (I), (II), (III) and (IV) in the polymer conjugate.

In an embodiment, at least one n in the recurring unit of formula (I) and the recurring unit of formula (II) is 1. In another embodiment, at least one n in the recurring unit of formula (I) and the recurring unit of formula (II) is 2.

In an embodiment, the amount of the agent, the percentage of the recurring unit of the formula (I) and the percentage of the recurring unit of the formula (II) in the polymer conjugate are selected to provide a polymer conjugate solubility that is greater than that of a comparable polyglutamic acid conjugate that comprises substantially the same amount of the agent. The range of pH values over which the polymer conjugate, comprising recurring units of the formula (I) and formula (II), has greater solubility than that of a comparable polyglutamic acid conjugate may be narrow or broad. As noted above, solubility is measured by forming a polymer conjugate solution comprising at least 5 mg/mL of the polymer conjugate in 0.9 wt. % aqueous NaCl at about 22°C, and determining the optical clarity. In an embodiment, the polymer conjugate is soluble over a pH range of at least about three pH units. In another embodiment, the polymer conjugate is soluble over a pH range of at least about 8 pH units. In another embodiment, the polymer conjugate is soluble over a pH range of at least about 9 pH units. In another embodiment, the pH range over which the polymer conjugate is soluble includes at least one pH value in the range of about 2 to about 5, e.g., at pH = 2, pH = 3, pH = 4 and/or pH = 5. Preferably, the pH range over which the polymer conjugate is soluble is broader than the pH range over which the comparable polyglutamic acid conjugate is soluble. For example, in an embodiment, the polymer conjugate is soluble over a pH range that is at least about one pH unit broader, preferably at least about two pH units broader, than the pH range over which the comparable polyglutamic acid conjugate is soluble.

The amount of polymer conjugate placed in solution to measure solubility can also vary greatly. In one embodiment, solubility is measured when the tested polymer conjugate solution comprises at least about 5 mg/mL of the polymer conjugate. In another embodiment, solubility is measured when the tested polymer conjugate solution comprises at least about 10 mg/mL of the polymer conjugate. In another embodiment, solubility is measured when the tested polymer conjugate solution comprises at least about 25 mg/mL of · the polymer conjugate. In another embodiment, solubility is measured when the tested polymer conjugate solution comprises at least about 100 mg/mL of the polymer conjugate. In another embodiment, solubility is measured when the tested polymer conjugate solution comprises at least about 150 mg/mL of the polymer conjugate. Those skilled in the art will understand that the comparable polyglutamic acid conjugate is tested at about the same concentration as that of the tested polymer conjugate.

Polymers comprising a recurring unit of the formula (I) and a recurring unit of the formula (II) may be prepared in various ways. In an embodiment, a polymeric reactant is dissolved or partially dissolved in a solvent to form a dissolved or partially dissolved polymeric reactant. The dissolved or partially dissolved polymeric reactant is then reacted with a second reactant and third reactant to form an intermediate product or, in some embodiments, a polymer comprising a recurring unit of the formula (I) and a recurring unit of the formula (II).

The polymeric reactant may comprise any suitable material capable of forming a polymer comprising a recurring unit of the formula (I) and a recurring unit of the formula (II). In an embodiment, the polymeric reactant comprises a recurring unit of the formula (V): 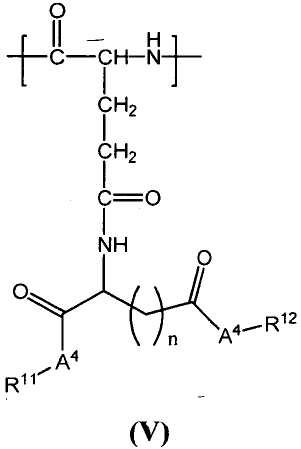 wherein each n is independently 1 or 2, each A⁴ is oxygen, and R¹¹ and R¹² are each independently selected from the group consisting of hydrogen, ammonium, and an alkali metal.

In an embodiment, the polymeric reactant may comprise a recurring unit of formula (VI): 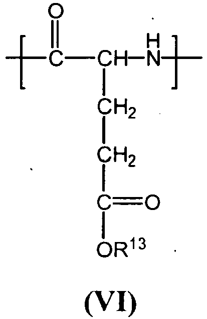 wherein R¹³ is hydrogen, ammonium, or an alkali metal.

The second reactant comprises an anticancer drug. In an embodiment, the second reactant may comprise a substituent. The substituent may be selected from the group consisting of hydroxy and an amine.

In an embodiment, the third reactant may comprise a substituent. The substituent may be selected from the group consisting of hydroxy and an amine. The third reactant comprises a compound that comprises an agent. For instance, the compound that comprises the agent may be selected from the group consisting of a targeting agent, an optical imaging agent, a magnetic resonance imaging agent and polyethylene glycol. In some embodiments, the third reactant comprises a polydentate polyethylene glycol ligand, a polydentate ligand precursor with protected oxygen atoms or a compound that includes an agent selected from the group consisting of a targeting agent, an optical imaging agent, a magnetic resonance imaging agent, and polyethylene glycol. In an embodiment, the agent included in the second reactant and the agent included in the reactant are not the same.

In an embodiment, the anticancer drug can be selected from the group consisting of a taxane, camptotheca, and anthracycline. In a preferred embodiment, the anticancer drug can comprise doxorubicin. In another preferred embodiment, the anticancer drug may comprise taxane, and the taxane may be selected from the group consisting of paclitaxel and docetaxel. Paclitaxel may be conjugated to the polymer in a number of ways. In an embodiment, paclitaxel is conjugated to the recurring unit of formula (I) at the oxygen atom attached to the C2'-carbon. In another embodiment, paclitaxel is conjugated to the recurring unit of formula (I) at the oxygen atom attached to the C7-carbon.. When the anticancer drug is a camptotheca, it is preferably camptothecin. In an embodiment when the anticancer drug is anthracycline, it can be doxorubicin,

The targeting agent is selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF), and an antibody. In an embodiment, the targeting agent interacts with a receptor selected from the group consisting of αᵥ,β₃-integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor. In some embodiments, the arginine-glycine-aspartate (RGD) peptide is cyclic(fKRGD).

In an embodiment, the optical imaging agent may be selected from the group consisting of an acridine dye, a coumarine dye, a rhodamine dye, a xanthene dye, a cyanine dye, and a pyrene dye.

In an embodiment, the compound that comprises the agent comprises a magnetic resonance imaging agent. In another embodiment, the magnetic resonance imaging agent comprises a paramagnetic metal compound. Preferably, the compound that comprises the agent comprises a Gd(III) compound. Exemplary, Gd(III) compounds include the following:

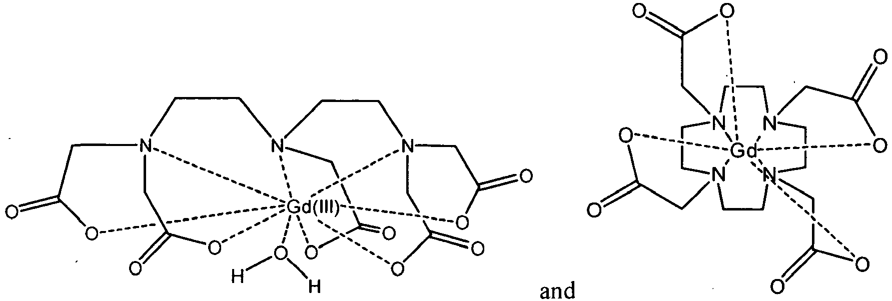

In an embodiment, a polydentate ligands may be conjugated to the polymer. Any suitable polydentate ligand may be used. In an embodiment, the polydentate ligand may be capable of reaction with a paramagnetic metal to form a magnetic resonance imaging agent. For example, the polydentate ligand may comprise several carboxylic acid and/or carboxylate groups. For example, polydentate ligands of the following structures may be conjugated to the polymer: 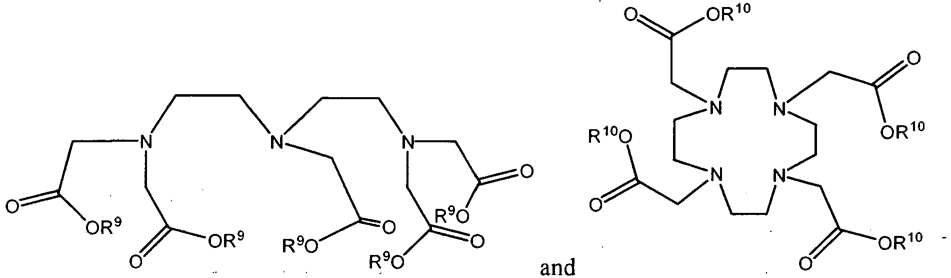 wherein each R⁹ and R¹⁰ are independently hydrogen, ammonium, or an alkali metal.

In another embodiment, a polydentate ligand precursor having protecting groups may be conjugated to the polymer. Such a precursor has its oxygen atoms protected by a suitable protecting group(s). Suitable protecting groups include lower alkyls, benzyls, and silyl groups. One example of a polydentate ligand precursor having protecting groups is provided as follows:

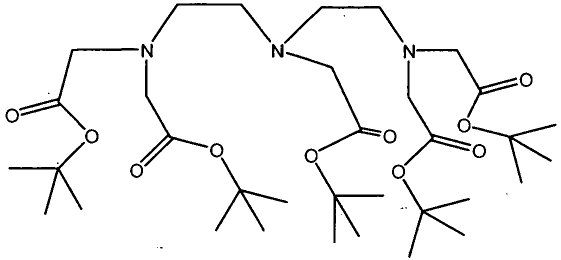

In some embodiments, the dissolved or partially dissolved polymer reactant is reacted with at least a portion of the second reactant before reacting with the third reactant. In an embodiment, the intermediate compound that forms after the addition of at least a portion of the second reactant can be isolated before adding the third reactant. In another embodiment, the third reactant can be added without isolating the intermediate compound that forms after the addition of the second reactant. In other embodiments, the dissolved or partially dissolved polymer reactant is reacted with at least a portion of the second reactant at about the same time as reacting with the third reactant. In an embodiment, the dissolved or partially dissolved polymer reactant is reacted with at least a portion of the third reactant before reacting with the second reactant.

In an embodiment, a method of making the polymer conjugate comprises reacting the dissolved or partially dissolved polymeric reactant with the second reactant and/or third reactant in the presence of a coupling agent. Any suitable coupling agent may be used. In an embodiment, the coupling agent is selected from the group consisting of 1-ethyl-3-(3-dimethylaminopropyl)-carbodiimide (EDC), 1,3-dicyclohexyl carbodiimide (DCC), 1,1'-carbonyl-diimidazole (CDI), N,N'-disuccinimidyl carbonate (DSC), N-[(dimethylamino)-1H-1,2,3-triazolo-[4,5-b]pyridine-1yl-methylene]-N-methylmethanaminium hexafluorophosphate N-oxide (HATU), 2-[(1H-benzotriazol-1-yl)-1,1,3,3-tetramethylaminium hexafluorophosphate (HBTU), 2-[(6-chloro-1H-benzotriazol-1-yl)-1,1,3,3-tetramethylaminium hexafluorophosphate (HCTU), benzotriazole-1-yl-oxy-tris-pyrrolidino-phosphonium hexafluorophosphate (PyBOP®), bromo-tris- pyrrolidino-phosphonium hexafluorophosphate (PyBroP®), 2-[(1H-benzotriazol-1-yl)-1,1,3,3-tetramethylaminium tetrafluoroborate (TBTU), and benzotriazol-1-yl-oxy-tris-(dimethylamino)phosphonium hexafluorophosphate (BOP).

Any suitable solvent that allows the reaction to take place may be used. In an embodiment, the solvent may be a polar aprotic solvent. For instance, the solvent may be selected from the group consisting of N,N-dimethylformamide (DMF), dimethyl sulfoxide (DMSO), N-methyl-2-pyridone (NMP), and N,N-dimethylacetamide (DMAc).

In another embodiment, the reaction may further comprise reacting the dissolved or partially dissolved polymeric reactant in the presence of a catalyst. Any catalyst that promotes the reaction may be used. In an embodiment, the catalyst may comprise 4-dimethylaminopyridine (DMAP).

In an embodiment, a polymer comprising a recurring unit of the formula (I) and a recurring unit of the formula (II) can be produced starting with polyglutamic acid and an amino acid such as asparatic and/or glutamic acid. Alternatively, in another embodiment, the polymer may be created by first converting the starting polyglutamic acid material into its salt form. The salt form of polyglutamic can be obtained by reacting polyglutamic acid with a suitable base, e.g., sodium bicarbonate. An amino acid moiety can be attached to the pendant carboxylic acid group of the polyglumatic acid. The weight average molecular weight of the polyglutamic acid may vary over a broad range, but is preferably from about 10,000 to about 500,000 daltons, and more preferably from about 25,000 to about 300,000 daltons. Such a reaction may be used to create poly-(γ-L-aspartyl-glutamine) or poly-(γ-L-glutamyl-glutamine).

In an embodiment, the amino acid is protected by a protecting group before attachment to the polyglutamic acid. One example of a protected amino acid moiety suitable for this reaction is L-aspartic acid di-t-butyl ester hydrochloride, shown below:

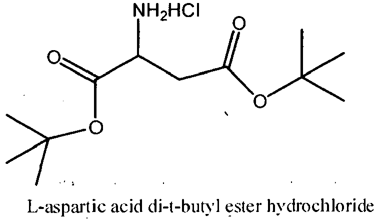

Reaction of the polyglutamic acid with the amino acid may take place in the presence of any suitable solvent. In an embodiment, the solvent can be an aprotic solvent. In a preferred embodiment, the solvent is N,N'-dimethylformamide.

In an embodiment, a coupling agent such as EDC, DCC, CDI, DSC, HATU, HBTU, HCTU, PyBOP®, PyBroP®, TBTU, and BOP can be used. In other embodiments, polyglutamic acid and an amino acid can be reacted using a catalyst (e.g., DMAP).

After completion of the reaction, if the oxygen atoms of the amino acid are protected, the protecting groups can be removed using known methods such as using a suitable acid (e.g., trifluoroacetic acid). If desired, the salt form of the polymer obtained from reacting polyglutamic acid with the amino acid can be formed by treating the acid form of the polymer with a suitable base solution, e.g., sodium bicarbonate solution.

The polymer may be recovered and/or purified by methods known to those skilled in the art. For example, the solvent may be removed by suitable methods, for instance, rotary evaporation. Additionally, the reaction mixture may be filtered into an acidic water solution to induce precipitation. The resultant precipitate can then be filtered, and washed with water.

In some embodiments, a polymer comprising a recurring unit of the formula (I) and a recurring unit of the formula (II) can also include a recurring unit of formula (III) as set forth above. One method for forming a polymer comprising recurring units of the formulae (I), (II), and (III) is by reacting a polymer comprising a recurring unit of formula (V), as described herein, with less than 1.0 equivalents of the polydentate ligand, the polydentate ligand with protected oxygen atoms, and/or a compound that comprises an agent based on the starting polymer. For example, in one embodiment, the polymer comprising a recurring unit of the formula (V) can be reacted with 0.25 equivalents of a targeting agent and 0.25 equivalents of a stabilizing agent to form a polymer comprising recurring units of formulae (I), (II) and (III).

In an embodiment, a polymer comprising a recurring unit of the formula (I) and a recurring unit of the formula (II) can also include a recurring unit of formula (IV) as set forth above. One method for forming a polymer comprising recurring units of the formulae (I), (II), and (IV) is by starting with polyglutamic acid and reacting it with an amino acid such as asparatic and/or glutamic acid, in an amount that is less than 1.0 equivalents of the amino acid based on polyglutamic acid. For example, in one embodiment, 0.7 equivalents of an amino acid based on the polyglutamic acid can be reacted with polyglutamic acid, so that about 70% of the recurring units of the resulting polymer comprise the amino acid. As discussed above, the oxygen atoms of the amino acid can be protected using a suitable protecting group. In an embodiment, the amino acid may be L-aspartic acid or L-glutamic acid. In another embodiment, the oxygen atoms of the amino acid can be protected with t-butyl groups. If the oxygen atoms of the amino acid are protected, the protecting groups can be removed using known methods such as a suitable acid (e.g., trifluoroacetic acid).

Conjugation of a group comprising an agent, a polydentate ligand, and/or a polydentate ligand precursor with protected oxygen atoms to the polymer acid or its salt form may be carried out in various ways, e.g., by covalently bonding the group comprising an agent, a polydentate ligand, and/or a polydentate ligand precursor with protected oxygen atoms to various polymers. One method for conjugating the aforementioned groups to the polymer obtained from polyglutamic acid and/or salt is by using heat (e.g, heat from using a microwave method). Alternatively, conjugation may take place at room temperature. Appropriate solvents, coupling agents, catalysts, and/or buffers as generally known to those skilled in the art and/or as described herein may be used to form the polymer conjugate. As with polyglutamic acid, both the salt or acid form of the polymer obtained from polyglutamic acid and/or salt and an amino acid can be used as starting material for forming the polymer conjugate.

Suitable agents that can be conjugated to the polymer obtained from polyglutamic acid and/or salt and an amino acid include drugs, optical agents, targeting agents, magnetic resonance imaging agents (e.g, paramagnetic metal compounds), stabilizing agents, polydentate ligands, and polydentate ligand precursors with protected oxygen atoms.

In one embodiment, the polymer obtained from polyglutamic acid and/or salt and an amino acid can be conjugated to an optical imaging agent such as those described herein. In an embodiment, the optical agent can be Texas Red-NH₂.

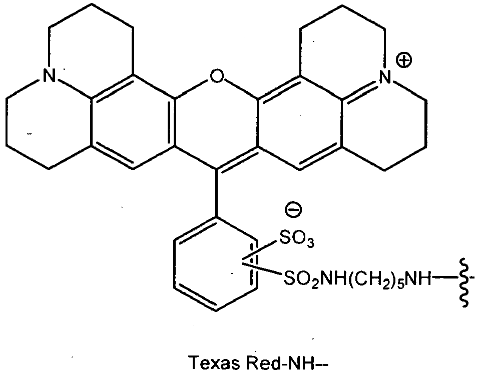

In one particular embodiment, a polymer comprising at least one recurring unit of the formula (I) and at least one recurring unit of the formula (II) may be reacted with DCC, Texas Red-NH₂ dye, pyridine, and 4-dimethylaminopyridine. The mixture is heated using a microwave method. In an embodiment, the reaction is heated up to a temperature in the range of about 100°-150°C. In another embodiment, the time the materials are heated ranges from 5 to 40 minutes. If desired, the reaction mixture can be cooled to room temperature. Suitable methods known to those skilled in the art can be used to isolate and/or purify the polymer conjugate. For instance, reaction mixture can be filtered into an acidic water solution. Any precipitate that forms can then be filtered and washed with water. Optionally, the precipitate can be purified by any suitable method. For example, the precipitate can be transferred into acetone and dissolved, and the resulting solution can be filtered again into a sodium bicarbonate solution. If desired, the resulting reaction solution can be dialyzed in water using a cellulose membrane and the polymer can be lyophilized and isolated.

In one embodiment, the polymer obtained from polyglutamic acid and/or salt and an amino acid can be conjugated to an anticancer drug. In an embodiment, the anticancer drug can be a taxane, camptotheca, and/or anthracycline. In an embodiment, the anticancer drug is a taxane such as paclitaxel or docetaxel. In other embodiments, the anticancer drug conjugated to the polymer is a camptotheca such as camptothecin. In some embodiments, the anticancer drug conjugated to the polymer is an anthracycline such as doxorubicin. In other embodiments, the anticancer drug conjugated to the polymer is paclitaxel. In an embodiment, paclitaxel may be joined to the polymer at the C2'-oxygen atom. In another embodiment, the paclitaxel may be joined to the polymer at the C7-oxygen atom. In another embodiment, the polymer comprises paclitaxel that is coupled to the polymer only by the C2'-oxygen atom. In still another embodiment, the polymer comprises paclitaxel that is coupled to the polymer only by the C7-oxygen atom. In yet another embodiment, the polymer comprises both C2'-conjugated paclitaxel groups and C7-conjugated paclitaxel groups.

The anti-cancer drug can be conjugated to the polymer obtained from polyglutamic acid and/or salt and an amino acid using the methods described above with respect to Texas-Red.

In an embodiment, paclitaxel, preferably in the presence of a coupling agent (e.g, EDC and/or DCC) and a catalyst (e.g, DMAP), can be reacted with the polymer obtained from polyglutamic acid and/or salt and an amino acid in a solvent (e.g, an aprotic solvent such as DMF). Additional agents, such as pyridine or hydroxybenzotriazole may be used. In one embodiment, the reaction may take place over the period of 0.5-2 days. Suitable methods known to those skilled in the art can be used to isolate and/or purify the polymer conjugate. For example, the reaction mixture can be poured into an acidic solution to form a precipitate. Any precipitate that forms can then be filtered and washed with water. Optionally, the precipitate can be purified by any suitable method. For example, the precipitate can be transferred into acetone and dissolved, and the resulting solution can be filtered again into a sodium bicarbonate solution. If desired, the resulting reaction solution can be dialyzed in water using a cellulose membrane and the polymer can be lyophilized and isolated. The content of paclitaxel in the resulting polymer may be determined by UV spectrometry.

Alternatively, the compound comprising the agent can be reacted with an amino acid such as glutamic and/or aspartic acid in which the compound comprising the agent is coupled (e.g., covalently bonded) to the amino acid. The amino acid-agent compound can then be reacted with polyglutamic acid or its salt to form the polymer conjugate. In one embodiment, paclitaxel is reacted with glutamic acid to form a compound in which the paclitaxel is covalently bonded to the pendant carboxylic acid group of the glutamic acid. The glutamic acid-paclitaxel compound can then be reacted with polyglutamic acid or its salt to form the polymer conjugate. In one embodiment, paclitaxel is reacted with aspartic acid to form a compound in which the paclitaxel is covalently bonded to the pendant carboxylic acid group of the aspartic acid. The aspartic acid-paclitaxel compound can then be reacted with polyglutamic acid or its salt to form the polymer conjugate. If desired, the paclitaxel coupled to the amino acid by the C2'-oxygen can be separated from the paclitaxel coupled to the amino acid by the C7-oxygen using known separation methods (e.g, HPLC).

After formation of the polymer conjugate, any free amount of agent not covalently bonded to the polymer may also be measured. For example, thin layer chromatography (TLC) may be used to confirm the substantial absence of free paclitaxel remaining in the compositions of polymers conjugated to paclitaxel.

In one embodiment, the polymer obtained from polyglutamic acid and/or salt and an amino acid can be conjugated to a polydentate ligand. Suitable polydentate ligands include diethylenetriaminepentacetic acid (DTPA), tetraazacyclododecane-1,4,7,10-tetraacetic acid (DOTA), (1,2-ethanediyldinitrilo)tetraacetate (EDTA), ethylenediamine, 2,2'-bipyridine (bipy), 1,10-phenanthroline (phen), 1,2-bis(diphenylphosphino)ethane (DPPE), 2,4-pentanedione (acac), and ethanedioate (ox). Appropriate solvents, coupling agents, catalysts, and/or buffers as generally known to those skilled in the art and/or described herein may be used to form the polymer conjugate. In another embodiment, the polymer obtained from polyglutamic acid and/or salt and an amino acid can be conjugated to a polydentate ligand precursor with protected oxygen atoms. As with polyglutamic acid, both the salt or acid form of the polymer obtained from polyglutamic acid and/or salt and an amino acid can be used as starting material for forming the polymer conjugate.

In an embodiment, the polydentate ligand is DTPA. In another embodiment, the polydentate ligand is DOTA. In one embodiment, the polydentate ligand such as DTPA (with or without protected oxygen atoms), preferably in the presence of a coupling agent (e.g, DCC) and a catalyst (e.g, DMAP), can be reacted with the polymer obtained from polyglutamic acid and/or salt and an amino acid in a solvent (e.g, an aprotic solvent such as DMF). If protecting groups are present, removal can achieved using suitable methods. For example, the polymer conjugate with the polydentate ligand precursor with protected oxygen atoms such as DTPA with oxygen atoms protected by t-butyl groups can be treated with acid such as trifluoroacetic acid. After removal of the protecting groups, the acid can be removed by rotary evaporation. In one embodiment, DTPA can be treated with a suitable base to remove the hydrogen atoms on the carboxylic acid -OH groups. In some embodiments, the base is sodium bicarbonate.

In one embodiment, the polymer obtained from polyglutamic acid and/or salt and an amino acid can be conjugated to a targeting agent selected from arginine-glycine-aspartate (RGD) peptides, fibronectin, folate, galactose, apolipoprotein, insulin, transferrin, fibroblast growth factors (FGF), epidermal growth factors (EGF), and antibodies. Targeting agents can also be chosen such that they interact with particular receptors. For example, a targeting agent can be chosen so that it interacts with one or more of the following receptors: αᵥ,β₃-integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor. In one embodiment, the arginine-glycine-aspartate (RGD) peptide is cyclic(fKRGD).

Both the salt or acid form of the polymer obtained from polyglutamic acid and/or salt and an amino acid can be used as starting material for forming the polymer conjugate with a targeting agent. In one embodiment, the targeting agent preferably in the presence of a coupling agent (e.g, DCC) and a catalyst (e.g, DMAP), can be reacted with the polymer obtained from polyglutamic acid and/or salt and an amino acid in a solvent (e.g_{;} an aprotic solvent such as DMF). After formation of the polymer conjugate, any free amount of agent not covalently bonded to the polymer may also be measured. For example, thin layer chromatography (TLC) may be used to confirm the substantial absence of any free targeting agent. Suitable methods known to those skilled in the art can be used to isolate and/or purify the polymer conjugate (e.g., lypholization).

In an embodiment, the polymer obtained from polyglutamic acid and/or salt and an amino acid can be conjugated to a magnetic resonance imaging agent. In an embodiment, the magnetic resonance imaging agent comprises a Gd(III) compound. One method for forming the magnetic resonance imaging agent is by reacting a paramagnetic metal with the polymer conjugate comprising a polydentate ligand. Suitable paramagnetic metals include Gd(III), Indium-111, and Yttrium-88. For example, a polymer conjugate comprising DTPA can be treated'with Gd(III) in a buffer solution for a period of several hours. Suitable methods known to those skilled in the art can be used to isolate and/or purify the polymer conjugate. For instance, the resulting reaction solution can be dialyzed in water using a cellulose membrane and the polymer can be lyophilized and isolated. The amount of paramagnetic metal may be quantified by inductively coupled plasma-optical emission spectroscopy (ICP-OES) measurement.

In one embodiment, the polymer obtained from polyglutamic acid and/or salt and an amino acid can be conjugated to the stabilizing agent, polyethylene glycol. In one method, the stabilizing agent, preferably in the presence of a coupling agent (e.g, DCC) and a catalyst (e.g, DMAP), can be reacted with the polymer obtained from polyglutamic acid and/or salt and an amino acid in a solvent (e.g, an aprotic solvent such as DMF). Progress of the reaction can be measured by any suitable method such as TLC. The resulting polymer conjugate can be purified using methods known to those skilled in the art such as dialysis.

Multiple compounds that comprise an agent can be conjugated to a polymer conjugate comprising a recurring unit of formula (I) and a recurring unit of formula (II). In some embodiments, the agents can be different. For example, a compound that comprises a targeting agent can be conjugated to a polymer comprising a recurring unit of formula (I) and a recurring unit of formula (II). The resulting polymer can then be reacted with a compound that comprises an imaging agent to form a polymer conjugate comprising a recurring unit of formula (I) and a recurring unit of formula (II) that includes both a targeting and an imaging agent. If desired, the polymer conjugate with a targeting and imaging agent can be further reacted with a compound comprising a stabilizing agent to thereby conjugate the stabilizing agent to the polymer.

The polymer conjugates may be used to deliver an imaging agent, targeting agent, magnetic resonance imaging agent and/or a drug to a selected tissue. For example, polymer conjugates comprising the Texas Red dye may be used to deliver an imaging agent to a selected tissue. In one embodiment, the polymer conjugates comprising a recurring unit of the formula (I) and a recurring unit of the formula (II) can be used to treat or ameliorate a disease or condition such as cancer. In an embodiment, the polymer conjugates described herein can be used to diagnose a disease or condition (e.g., cancer). In yet one more embodiment, the polymer conjugates described herein can be used to image a portion of tissue. In some embodiments, the disease or condition can be a cancer such as lung cancer, breast cancer, colon cancer, ovarian cancer, prostate cancer, and melanoma. In an embodiment, the disease or condition can be a tumor selected from the group consisting of lung tumor, breast tumor, colon tumor, ovarian tumor, prostate tumor, and melanoma tumor.

The polymers described above may be formed into nanoparticles in aqueous solution. Conjugates comprising a polymer and a drug may be formed into nanoparticles in a similar manner. Such nanoparticles may be used to preferentially deliver a drug to a selected tissue.

### Pharmaceutical Compositions

In some embodiments, prodrugs, metabolites, stereoisomers, hydrates, solvates, polymorphs, and pharmaceutically acceptable salts of the compounds disclosed herein (e.g., the polymer conjugate and/or the agent that it comprises) are provided.

A "prodrug" refers to an agent that is converted into the parent drug *in vivo*. Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. An example, without limitation, of a prodrug would be a compound which is administered as an ester (the "prodrug") to facilitate transmittal across a cell membrane where water solubility is detrimental to mobility but which then is metabolically hydrolyzed to the carboxylic acid, the active entity, once inside the cell where water-solubility is beneficial. A further example of a prodrug might be a short peptide (polyaminoacid) bonded to an acid group where the peptide is metabolized to reveal the active moiety. Conventional procedures for the selection and preparation of suitable prodrug derivatives are described, for example, in Design of Prodrugs, (ed. H. Bundgaard, Elsevier, 1985).

The term "pro-drug ester" refers to derivatives of the compounds disclosed herein formed by the addition of any of several ester-forming groups that are hydrolyzed under physiological conditions. Examples of pro-drug ester groups include pivaloyloxymethyl, acetoxymethyl, phthalidyl, indanyl and methoxymethyl, as well as other such groups known in the art, including a (5-R-2-oxo-1,3-dioxolen-4-yl)methyl group. Other examples of pro-drug ester groups can be found in, for example, T. Higuchi and V. Stella, in "Pro-drugs as Novel Delivery Systems", Vol. 14, A.C.S. Symposium Series, American Chemical Society (1975); and "Bioreversible Carriers in Drug Design: Theory and Application", edited by E. B. Roche, Pergamon Press: New York, 14-21 (1987) (providing examples of esters useful as prodrugs for compounds containing carboxyl groups).

The term "pharmaceutically acceptable salt" refers to a salt of a compound that does not cause significant irritation to an organism to which it is administered and does not abrogate the biological activity and properties of the compound. In some embodiments, the salt is an acid addition salt of the compound. Pharmaceutical salts can be obtained by reacting a compound with inorganic acids such as hydrohalic acid (e.g., hydrochloric acid or hydrobromic acid), sulfuric acid, nitric acid, phosphoric acid. Pharmaceutical salts can also be obtained by reacting a compound with an organic acid such as aliphatic or aromatic carboxylic or sulfonic acids, for example acetic, succinic, lactic, malic, tartaric, citric, ascorbic, nicotinic, methanesulfonic, ethanesulfonic, p-toluensulfonic, salicylic or naphthalenesulfonic acid. Pharmaceutical salts can also be obtained by reacting a compound with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, C₁-C₇ alkylamine, cyclohexylamine, triethanolamine, ethylenediamine, and salts with amino acids such as arginine, lysine.

If the manufacture of pharmaceutical formulations involves intimate mixing of the pharmaceutical excipients and the active ingredient in its salt form, then it may be desirable to use pharmaceutical excipients which are non-basic, that is, either acidic or neutral excipients.

In various embodiments, the compounds disclosed herein (e.g., the polymer conjugate and/or the agent that it comprises) can be used alone, in combination with other compounds disclosed herein, or in combination with one or more other agents active in the therapeutic areas described herein.

In another aspect, the present disclosure relates to a pharmaceutical composition comprising one or more physiologically acceptable surface active agents, carriers, diluents, excipients, smoothing agents, suspension agents, film forming substances, and coating assistants, or a combination thereof; and a compound (e.g., the polymer conjugate and/or the agent that it comprises) disclosed herein. Acceptable carriers or diluents for therapeutic use are well known in the pharmaceutical art, and are described, for example, in Remington's Pharmaceutical Sciences, 18th Ed., Mack Publishing Co., Easton, PA (1990) . Preservatives, stabilizers, dyes, sweeteners, fragrances, flavoring agents may be provided in the pharmaceutical composition. For example, sodium benzoate, ascorbic acid and esters of p-hydroxybenzoic acid may be added as preservatives. In addition, antioxidants and suspending agents may be used. In various embodiments, alcohols, esters, sulfated aliphatic alcohols may be used as surface active agents; sucrose, glucose, lactose, starch, crystallized cellulose, mannitol, light anhydrous silicate, magnesium aluminate, magnesium metasilicate aluminate, synthetic aluminum silicate, calcium carbonate, sodium acid carbonate, calcium hydrogen phosphate, calcium carboxymethyl cellulose may be used as excipients; magnesium stearate, talc, hardened oil may be used as smoothing agents; coconut oil, olive oil, sesame oil, peanut oil, soya may be used as suspension agents or lubricants; cellulose acetate phthalate as a derivative of a carbohydrate such as cellulose or sugar, or methylacetate-methacrylate copolymer as a derivative of polyvinyl may be used as suspension agents; and plasticizers such as ester phthalates may be used as suspension agents.

The term "pharmaceutical composition" refers to a mixture of a compound disclosed herein (e.g., the polymer conjugate and/or the agent that it comprises) with other chemical components, such as diluents or carriers. The pharmaceutical composition facilitates administration of the compound to an organism. Multiple techniques of administering a compound exist in the art including oral, injection, aerosol, parenteral, and topical administration. Pharmaceutical compositions can also be obtained by reacting compounds with inorganic or organic .acids such as hydrochloric acid, hydrobromic acid, sulfuric acid, nitric acid, phosphoric acid, methanesulfonic acid, ethanesulfonic acid, p-toluenesulfonic acid, salicylic acid

The term "carrier" refers to a chemical compound that facilitates the incorporation of a compound into cells or tissues. For example dimethyl sulfoxide (DMSO) is a commonly utilized carrier as it facilitates the uptake of many organic compounds into the cells or tissues of an organism.

The term "diluent" refers to chemical compounds diluted in water that will dissolve the compound of interest (e.g., the polymer conjugate and/or the agent that it comprises) as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound. The term "physiologically acceptable" refers to a carrier or diluent that does not abrogate the biological activity and properties of the compound.

The pharmaceutical compositions described herein can be administered to a human patient *per se,* or in pharmaceutical compositions where they are mixed with other active ingredients, as in combination therapy, or suitable carriers or excipient(s). Techniques for formulation and administration of the compounds of the instant application may be found in "Remington's Pharmaceutical Sciences," Mack Publishing Co., Easton, PA, 18th edition, 1990.

Suitable routes of administration may, for example, include oral, rectal, transmucosal, topical, or intestinal administration; parenteral delivery, including intramuscular, subcutaneous, intravenous, intramedullary injections, as well as intrathecal, direct intraventricular, intraperitoneal, intranasal, or intraocular injections. The compounds (e.g., the polymer conjugate and/or the agent that it comprises) can also be administered in sustained or controlled release dosage forms, including depot injections, osmotic pumps, pills, transdermal (including electrotransport) patches for prolonged and/or timed, pulsed administration at a predetermined rate.

The pharmaceutical compositions of the present invention may be manufactured in a manner that is itself known, *e.g.,* by means of conventional mixing, dissolving, granulating, dragee-making, levigating, emulsifying, encapsulating, entrapping or tabletting processes.

Pharmaceutical compositions for use in accordance with the present invention thus may be formulated in conventional manner using one or more physiologically acceptable carriers comprising excipients and auxiliaries which facilitate processing of the active compounds into preparations which can be used pharmaceutically. Proper formulation is dependent upon the route of administration chosen. Any of the well-known techniques, carriers, and excipients may be used as suitable and as understood in the art; e.g., in Remington's Pharmaceutical Sciences, above.

Injectables can be prepared in conventional forms, either as liquid solutions or suspensions, solid forms suitable for solution or suspension in liquid prior to injection, or as emulsions. Suitable excipients are, for example, water; saline, dextrose, mannitol, lactose, lecithin, albumin, sodium glutamate, cysteine hydrochloride. In addition, if desired, the injectable pharmaceutical compositions may contain minor amounts of nontoxic auxiliary substances, such as wetting agents, pH buffering agents. Physiologically compatible buffers include Hanks's solution, Ringer's solution, or physiological saline buffer. If desired, absorption enhancing preparations (for example, liposomes), may be utilized.

For transmucosal administration, penetrants appropriate to the barrier to be permeated may be used in the formulation.

Pharmaceutical formulations for parenteral administration, *e.g.,* by bolus injection or continuous infusion, include aqueous solutions of the active compounds in water-soluble form. Additionally, suspensions of the active compounds may be prepared as appropriate oily injection suspensions. Suitable lipophilic solvents or vehicles include fatty oils such as sesame oil, or other organic oils such as soybean, grapefruit or almond oils, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g*., sterile pyrogen-free water, before use.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions, for oral ingestion by a patient to be treated. Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, *e.g.,* dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch.

Further disclosed herein are various pharmaceutical compositions well known in the pharmaceutical art for uses that include intraocular, intranasal, and intraauricular delivery. Suitable penetrants for these uses are generally known in the art. Pharmaceutical compositions for intraocular delivery include aqueous ophthalmic solutions of the active compounds in water-soluble form, such as eyedrops, or in gellan gum (Shedden et al., Clin. Ther., 23(3):440-50 (2001)) or hydrogels (Mayer et al., Ophthalmologica, 210(2):101-3 (1996)); ophthalmic ointments; ophthalmic suspensions, such as microparticulates, drug-containing small polymeric particles that are suspended in a liquid carrier medium (Joshi, A., J. Ocul. Pharmacol., 10(1):29-45 (1994)), lipid-soluble formulations (Alm et al., Prog. Clin. Biol. Res., 312:447-58 (1989)), and microspheres (Mordenti, Toxicol. Sci., 52(1):101-6 (1999)); and ocular inserts. Such suitable pharmaceutical formulations are most often and preferably formulated to be sterile, isotonic and buffered for stability and comfort. Pharmaceutical compositions for intranasal delivery may also include drops and sprays often prepared to simulate in many respects nasal secretions to ensure maintenance of normal ciliary action. As disclosed in Remington's Pharmaceutical Sciences, 18th Ed., Mack Publishing Co., Easton, PA (1990), and well-known to those skilled in the art, suitable formulations are most often and preferably isotonic, slightly buffered to maintain a pH of 5.5 to 6.5, and most often and preferably include antimicrobial preservatives and appropriate drug stabilizers. Pharmaceutical formulations for intraauricular delivery include suspensions and ointments for topical application in the ear. Common solvents for such aural formulations include glycerin and water.

The compounds may also be formulated in rectal compositions such as suppositories or retention enemas, *e.g.,* containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For hydrophobic compounds, a suitable pharmaceutical carrier may be a cosolvent system comprising benzyl alcohol, a nonpolar surfactant, a water-miscible organic polymer, and an aqueous phase. A common cosolvent system used is the VPD co-solvent system, which is a solution of 3% w/v benzyl alcohol, 8% w/v of the nonpolar surfactant Polysorbate 80™, and 65% w/v polyethylene glycol 300, made up to volume in absolute ethanol. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of POLYSORBATE 80™; the fraction size of polyethylene glycol may be varied; other biocompatible polymers may replace polyethylene glycol, *e.g.,* polyvinyl pyrrolidone; and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few hours or weeks up to over 100 days. Depending on the chemical nature and the biological stability of the therapeutic reagent, additional strategies for protein stabilization may be employed.

Agents intended to be administered intracellularly may be administered using techniques well known to those of ordinary skill in the art. For example, such agents may be encapsulated into liposomes. All molecules present in an aqueous solution at the time of liposome formation are incorporated into the aqueous interior. The liposomal contents are both protected from the external micro-environment and, because liposomes fuse with cell membranes, are efficiently delivered into the cell cytoplasm. The liposome may be coated with a tissue-specific antibody. The liposomes will be targeted to and taken up selectively by the desired organ. Alternatively, small hydrophobic organic molecules may be directly administered intracellularly.

Additional therapeutic or diagnostic agents may be incorporated into the pharmaceutical compositions. Alternatively or additionally, pharmaceutical compositions may be combined with other compositions that contain other therapeutic or diagnostic agents.

### Methods of Administration

The compounds or pharmaceutical compositions may be administered to the patient by any suitable means. Non-limiting examples of methods of administration include, among others, (a) administration though oral pathways, which administration includes administration in capsule, tablet, granule, spray, syrup, or other such forms; (b) administration through non-oral pathways such as rectal, vaginal, intraurethral, intraocular, intranasal, or intraauricular, which administration includes administration as an aqueous suspension, an oily preparation or as a drip, spray, suppository, salve, ointment; (c) administration via injection, subcutaneously, intraperitoneally, intravenously, intramuscularly, intradermally, intraorbitally, intracapsularly, intraspinally, intrasternally, including infusion pump delivery; (d) administration locally such as by injection directly in the renal or cardiac area, e.g., by depot implantation; as well as (e) administration topically; as deemed appropriate by those of skill in the art for bringing the active compound into contact with living tissue.

Pharmaceutical compositions suitable for administration include compositions where the active ingredients are contained in an amount effective to achieve its intended purpose. The therapeutically effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize. More specifically, a therapeutically effective amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein.

As will be readily apparent to one skilled in the art, the useful *in vivo* dosage to be administered and the particular mode of administration will vary depending upon the age, weight and mammalian species treated, the particular compounds employed, and the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods.

In non-human animal studies, applications of potential products are commenced at higher dosage levels, with dosage being decreased until the desired effect is no longer achieved or adverse side effects disappear. The dosage may range broadly, depending upon the desired effects and the therapeutic indication. Typically, dosages may be between about 10 microgram/kg and 100 mg/kg body weight, preferably between about 100 microgram/kg and 10 mg/kg body weight. Alternatively dosages may be based and calculated upon the surface area of the patient, as understood by those of skill in the art.

The exact formulation, route of administration and dosage for the pharmaceutical compositions of the present invention can be chosen by the individual physician in view of the patient's condition. (See e.g., Fingl et al. 1975, in "The Pharmacological Basis of Therapeutics" .Typically, the dose range of the composition administered to the patient can be from about 0.5 to 1000 mg/kg of the patient's body weight. The dosage may be a single one or a series of two or more given in the course of one or more days, as is needed by the patient. In instances where human dosages for compounds have been established for at least some condition, the present invention will use those same dosages, or dosages that are between about 0.1% and 500%, more preferably between about 25% and 250% of the established human dosage. Where no human dosage is established, as will be the case for newly-discovered pharmaceutical compositions, a suitable human dosage can be inferred from ED₅₀ or ID₅₀ values, or other appropriate values derived from *in vitro* or *in vivo* studies, as qualified by toxicity studies and efficacy studies in animals.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Although the exact dosage will be determined on a drug-by-drug basis, in most cases, some generalizations regarding the dosage can be made. The daily dosage regimen for an adult human patient may be, for example, an oral dose of between 0.1 mg and 2000 mg of each active ingredient, preferably between 1 mg and 500 mg, e.g. 5 to 200 mg. In other embodiments, an intravenous, subcutaneous, or intramuscular dose of each active ingredient of between 0.01 mg and 100 mg, preferably between 0.1 mg and 60 mg, e.g. 1 to 40 mg is used. In cases of administration of a pharmaceutically acceptable salt, dosages may be calculated as the free base. In some embodiments, the composition is administered 1 to 4 times per day. Alternatively the compositions of the invention may be administered by continuous intravenous infusion, preferably at a dose of each active ingredient up to 1000 mg per day. As will be understood by those of skill in the art, in certain situations it may be necessary to administer the compounds disclosed herein in amounts that exceed, or even far exceed, the above-stated, preferred dosage range in order to effectively and aggressively treat particularly aggressive diseases or infections. In some embodiments, the compounds will be administered for a period of continuous therapy, for example for a week or more, or for months or years.

Dosage amount and interval may be adjusted individually to provide plasma levels of the active moiety which are sufficient to maintain the modulating effects, or minimal effective concentration (MEC). The MEC will vary for each compound but can be estimated from in vitro data. Dosages necessary to achieve the MEC will depend on individual characteristics and route of administration. However, HPLC assays or bioassays can be used to determine plasma concentrations.

Dosage intervals can also be determined using MEC value. Compositions should be administered using a regimen which maintains plasma levels above the MEC for 10-90% of the time, preferably between 30-90% and most preferably between 50-90%.

In cases of local administration or selective uptake, the effective local concentration of the drug may not be related to plasma concentration.

The amount of composition administered may be dependent on the subject being treated, on the subject's weight, the severity of the affliction, the manner of administration and the judgment of the prescribing physician.

Compounds disclosed herein (e.g., the polymer conjugate and/or the agent that it comprises) can be evaluated for efficacy and toxicity using known methods. For example, the toxicology of a particular compound, or of a subset of the compounds, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular compound may be established using several recognized methods, such as *in vitro* methods, animal models, or human clinical trials. Recognized *in vitro* models exist for nearly every class of condition, including cancer, cardiovascular disease, and various immune dysfunction. Similarly, acceptable animal models may be used to establish efficacy of chemicals to treat such conditions. When selecting a model to determine efficacy, the skilled artisan can be guided by the state of the art to choose an appropriate model, dose, and route of administration, and regime. Of course, human clinical trials can also be used to determine the efficacy of a compound in humans.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions comprising a compound of the invention formulated in a compatible pharmaceutical carrier may also be prepared, placed in an appropriate container, and labeled for treatment of an indicated condition.

### EXAMPLES

### Materials

Poly-L-glutamate sodium salts with different molecular weights (average molecular weights of 41,400 (PGA(97k)), 17,600 (PGA(44k)), 16,000 (PGA(32k)), and 10,900 (PGA(21k)) daltons based on multi-angle light scattering (MALS)); N-(3-dimethylaminopropyl)-N'-ethylcarbodiimide hydrochloride (EDC); hydroxybenzotriazole (HOBt); pyridine; 4-dimethylaminopyridine (DMAP); N,N'-dimethylformamide (DMF); gadolinium-acetate; chloroform; camptothecin, and sodium bicarbonate were purchased from Sigma-Aldrich Chemical company. Poly-L-glutamate was converted into poly-L-glutamic acid using 2 N hydrochloric acid solution. Trifluoroacetic acid (TFA) was purchased from Bioscience. L-glutamic acid di-t-butyl ester hydrochloride (H-Glu(OtBu)-OtBu·HCl), N-α-CBZ-L-glutamic acid α-benzyl ester (Z-Glu-OBzl) were purchased from Novabiochem (La Jolla, CA). Paclitaxel and doxorubicin was purchased from PolyMed (Houston, TexasThe chemical p-NH₂-Bn-DPTA-penta-(*t*.-Bu ester) was purchased from Macrocyclics (Dallas, Texas). ¹H NMR was obtained from Joel (400 MHz), and particle sizes were measured by ZetalPals (Brookhaven Instruments Corporation). Microwave chemistry was carried out in Biotage. Molecular weights of polymers were determined by size exclusion chromatography (SEC) combined with a multi-angle light scattering (MALS) (Wyatt Corporation) detector:

A poly-(γ-L-glutamyl-glutamine) were prepared from a polyglutamate sodium salt, according to the procedures described in U.S. Patent Application 11/566,141, filed December 1, 2006 . U.S. Patent Application 11/566,141 describes the synthesis of the polymer described therein (e.g, poly-(γ-L-glutamyl-glutamine), poly-(γ-L-aspartyl-glutamine), poly-(γ-L-glutamyl-glutamine)-poly-L-glutamic acid, and poly-(y-L- aspartyl-glutamine)-poly-L-glutamic acid. Average molecular weights of the polymers were determined using the system and conditions described below (hereinafter, referred to as the Heleos system with MALS detector).

### SEC-MALS Analysis Conditions:

| | |
|---|---|
| ■ HPLC system: | Agilent 1200 |
| ■ Column: | Shodex SB 806M HQ |
| | (exclusion limit for Pullulan is 20,000,000, particle size: 13 micron, size (mm) IDxLength; 8.0 x300) |
| ■ Mobile Phase: | 1xDPBS or 1% LiBr in DPBS (pH7.0) |
| ■ Flow Rate: | 1 ml/min |
| ■ MALS detector: | DAWN HELEOS from Wyatt |
| ■ DRI detector: | Optilab rEX from Wyatt |
| ■ On-line Viscometer: | ViscoStar from Wyatt |
| ■ Software: | ASTRA 5.1.9 from Wyatt |
| ■ Sample Concentration: | 1-2 mg/ml |
| ■ Injection volume: | 100 µl |

dn/dc value of polymer: 0.185 was used in the measurement.
BSA was used as a control before actual samples are run.

Synthesis of fK(CBZ)R(Pdf)GD(OtBu)-protected, (fKRGD-protected), was carried out by a standard Fmoc-solid phase using 2-chlorotrityl chloride resin, HBTU and HOBt coupling agents with diisopropylethylamine (DIPEA). Deprotection of Fmoc group was carried out in 20% piperidine in DMF. Cleavage of fKRGD-protected from the resin was carried out in acetic acid: trifluoroethanol:dichloromethane (1:1:3). Cyclization of fKRGD-protected was carried out using NaHCO₃ and DPPA in DMF. Deprotection of the CBZ group was carried out under a hydrogen atmosphere in methanol with 10% Pd/C catalyst. Deprotection of cyclic(fKRGD) was carried out in 95% TFA. Purification of cyclic(fKRGD)-protected and cyclic(fKRGD) was carried out in HPLC system and purity of the products were confirmed with LC-MS.

### EXAMPLE 1

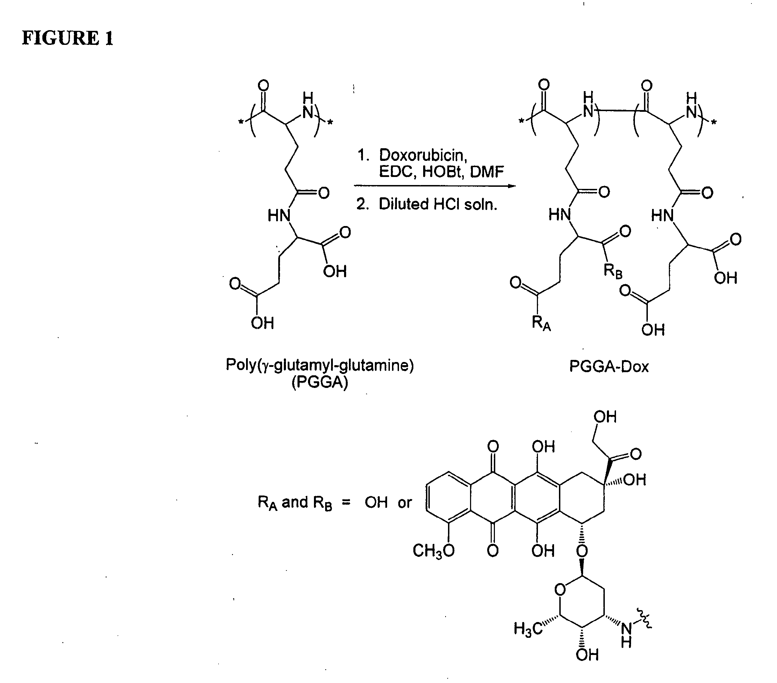

A PGGA-Dox polymer conjugate was prepared according to the general scheme illustrated in Figure 1 as follows:

Poly-(γ-L-glutamyl-glutamine) (150 mg) was dissolved in DMF (15 mL). Doxorubicin (25 mg), EDC (50 mg), and HOBt (50 mg) were added. The mixture was stirred for 24 hours. The reaction went to completion based on absence of free doxorubicin as determined by thin layer chromatography (TLC). Diluted HCl solution (0.2M) was added to induce precipitation. The mixture was then stirred for 2 minutes and centrifuged at 10,000 rpm for 15 minutes. The resulting solid precipitate was collected and freeze-dried. The product, PGGA-Dox, (130 mg) was obtained and was confirmed by ¹H-NMR.

### EXAMPLE 2

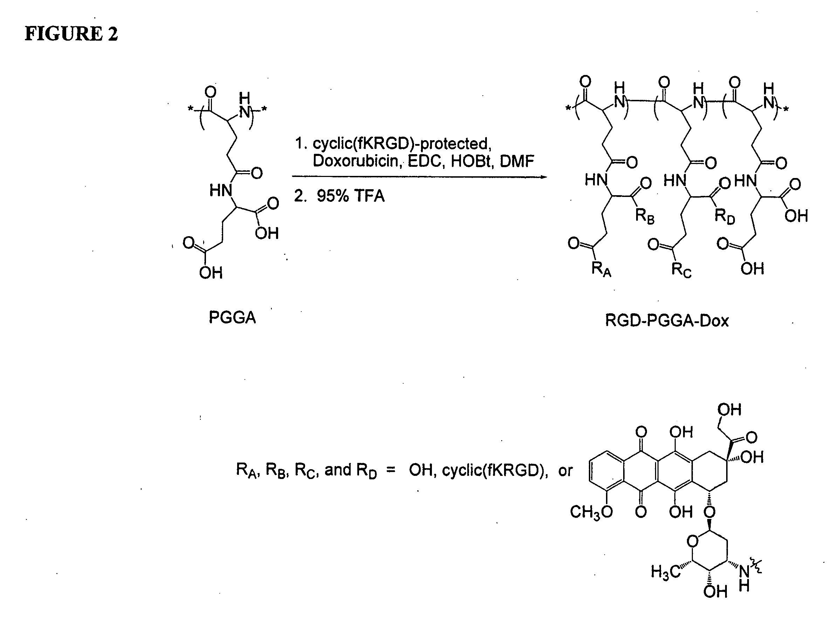

A RGD-PGGA-Dox polymer conjugate was prepared according to the general scheme illustrated in Figure 2 as follows:

Poly-(γ-L-glutamyl-glutamine), (PGGA), (100 mg) was dissolved in DMF (6 mL). Doxorubicin (20 mg), cyclic(fKRGD) (15 mg), EDC (56 mg), and HOBt (40 mg) were added. The mixture was stirred for 24 hours. The reaction went to completion based on absence of free doxorubicin as determined by thin layer chromatography (TLC). Diluted HCl solution (0.2M) was added to induce precipitation. The mixture was then stirred for 2 minutes and centrifuged at 10,000 rpm for 15 minutes. The resulting solid precipitate was collected, washed with water, and freeze-dried. The product, (cyclic(fKRGD))-PGGA-Dox, (75 mg) was obtained and was confirmed by ¹H-NMR.

### EXAMPLE 3

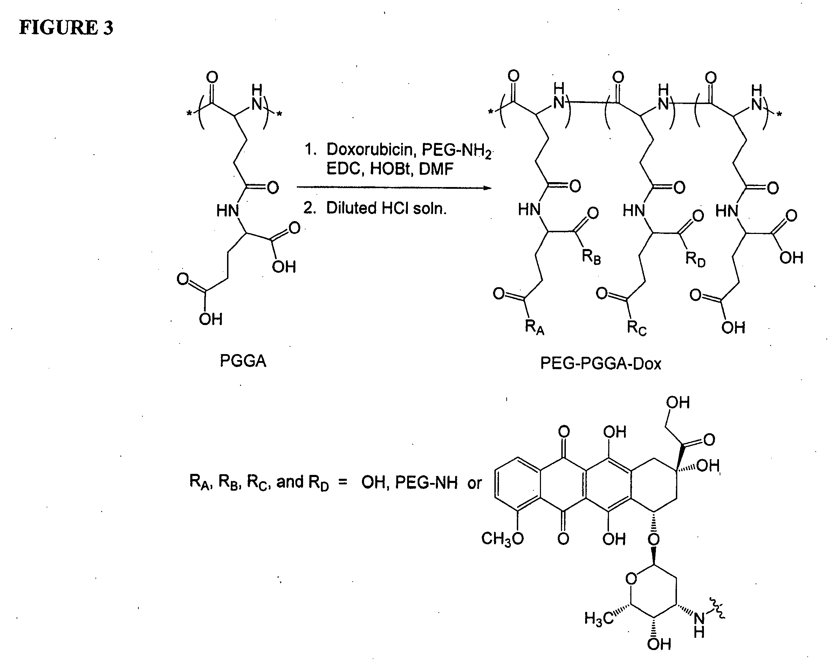

A PEG-PGGA-Dox polymer conjugate was prepared according to the general scheme illustrated in Figure 3 as follows:

Poly-(γ-L-glutamyl-glutamine) (420 mg) was dissolved in DMF (40 mL). EDC (100 mg) and HOBt (100 mg) were added. A solution of doxorubicin (47 mg) and polyethylene glycol, (PEG)-NH₂, (55 mg) in DMF (3 mL) were added. DMF (3 mL) was added. The mixture was stirred for 24 hours. The reaction went to completion based on absence of free doxorubicin and PEG-NH₂ as determined by thin layer chromatography (TLC). Diluted HCl solution (0.2M) was added and the mixture was dialyzed for 24 hours in water (4L x 5 times). After lyophilization, the product, PEG-PGGA-Dox, was obtained (366 mg), and confirmed by ¹H-NMR.

### EXAMPLE 4

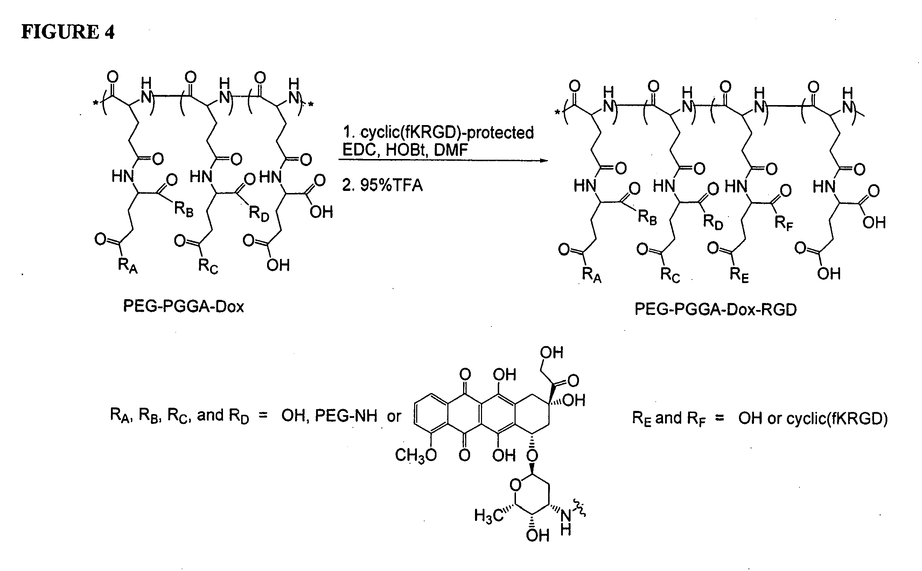

A PEG-PGGA-Dox-RGD polymer conjugate was prepared according to the general scheme illustrated in Figure 4 as follows:

PEG-PGGA-Dox (150 mg) was dissolved in DMF (15 mL). Cyclic(fKRGD) (20 mg), EDC (50 mg), and HOBt (50 mg) were added. The mixture was stirred for 24 hours. Diluted HCl solution (0.2M) was added to induce precipitation. The mixture was then stirred for 2 minutes and centrifuged at 10,000 rpm for 15 minutes. A solid precipitate was collected, washed with water, and freeze-dried. The solid was then treated with 95% TFA for 4 hours. TFA was removed by rotary evaporation and the mixture was basified with sodium bicarbonate solution and dialyzed in water for 24 hours. The product, PEG-PGGA-Dox-(cyclic(fKRGD)), was freeze-dried. The product (120 mg) was confirmed by ¹H-NMR.

### EXAMPLE 5

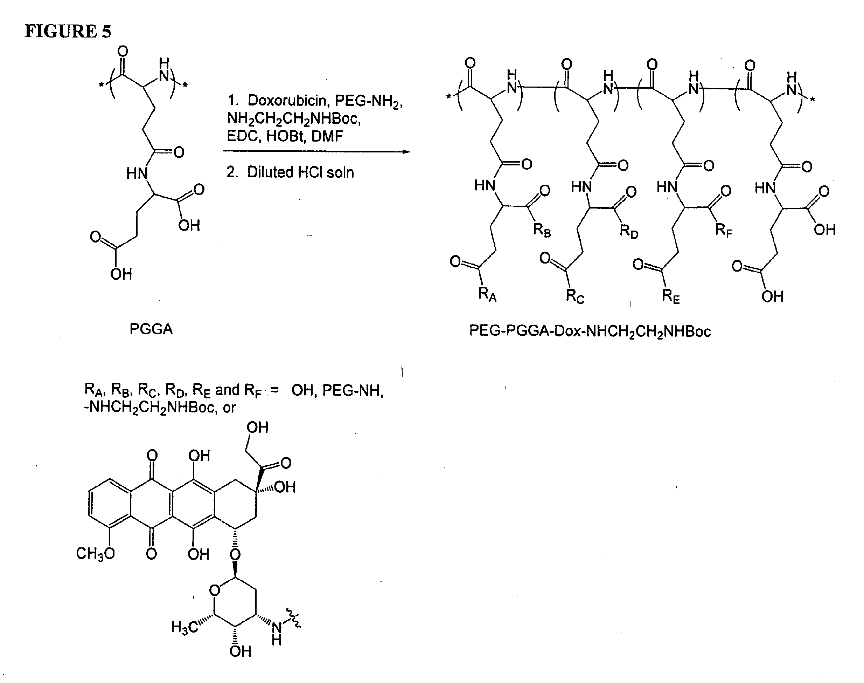

A PEG-PGGA-Dox-NHCH₂CH₂NHBoc polymer conjugate was prepared according to the general scheme illustrated in Figure 5 as follows:

Poly-(γ-L-glutamyl-glutamine) (250mg) was dissolved in DMF (30 mL). EDC (50 mg) and HOBt (50 mg) were added. A solution of doxorubicin (27 mg), NH₂CH₂CH₂NHBoc (10 mg) and PEG-NH₂ (27 mg) in DMF (3 mL) were added. The mixture was stirred for 24 hours. The reaction went to completion based on absence of free doxorubicin, PEG-NH₂, and NH₂CH₂CH₂NHBoc as determined by thin layer chromatography (TLC). Diluted HCl solution (0.2M) was then added and the mixture was dialyzed for 24 hours in water (4L x 5 times). The product was freeze-dried. The product, PEG-PGGA-Dox-N-HCH₂CH₂N-HBoc, (180 mg) was collected and confirmed by ¹H-NMR.

### EXAMPLE 6

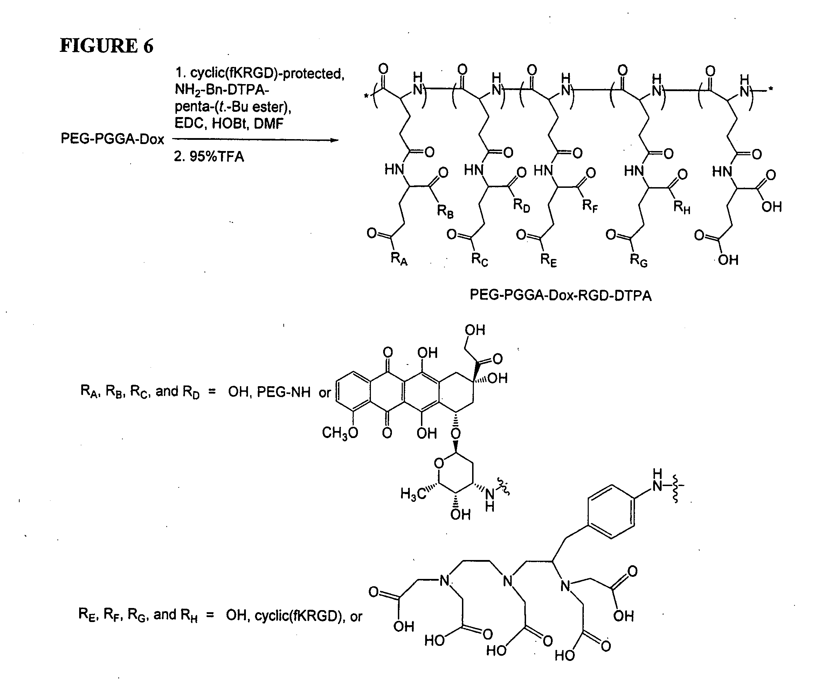

A PEG-PGGA-Dox-RGD-DTPA polymer conjugate was prepared according to the general scheme illustrated in Figure 6 as follows:

PEG-PGGA-Dox (150 mg) was dissolved in DMF (15 mL). Cyclic(fKRGD) (20 mg), EDC (50 mg), and HOBt (50 mg) were added. The mixture was stirred for 24 hours. Diluted HCl solution (0.2M) was then added to induce precipitation. The mixture was stirred for 2 minutes and centrifuged at 10,000 rpm for 15 minutes. A solid precipitate was collected, washed with water, and freeze-dried. The resulting solid was treated with 95% TFA for 4 hours. TFA was removed by rotary evaporation and the mixture was dialyzed in water for 24 hours. The product, PEG-PGGA-Dox-(cyclic(fKRGD))-DTPA, was freeze-dried. The product was confirmed by ^{I}H-NMR.

### EXAMPLE 7

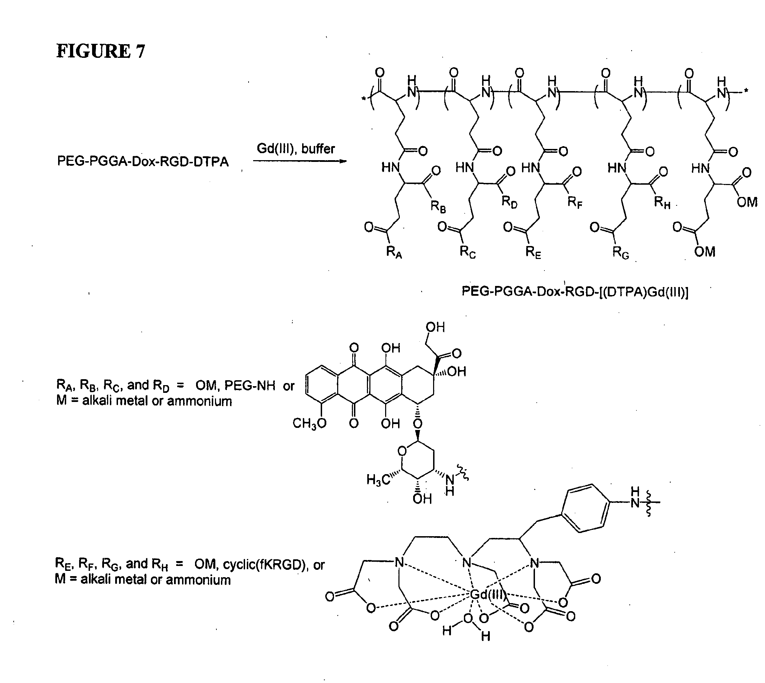

A PEG-PGGA-Dox-RGD-[(DTPA)Gd(III)] polymer conjugate was prepared according to the general scheme illustrated in Figure 7 as follows:

PEG-PGGA-Dox-(cyclic(fKRGD))-DTPA (45 mg) was dissolved in EDTA buffers (10 mL). A solution of Gd(III) (5 mg) in EDTA (1 mL) was added. The mixture was stirred for 4 hours and poured into sodium bicarbonate solution (50 mL) and dialyzed in water. The product, PEG-PGGA-Dox-(cyclic(fKRGD))-[(DTPA)Gd(III)], was lyophilized.

### EXAMPLE 8

### Cell culture and preparation:

B16F0 cells were purchased from ATCC (CRL-6322, ATCC American Type Culture Collection, Rockville, MD) and were grown in Dulbecco's modified Eagle's medium (DMEM) with 10% fetal bovine serum and 100 units/mL penicillin. The cells were grown at 37°C in 5% CO₂ environment. The culture medium was removed and discarded. The cells were rinsed with Dulbecco Phosphate Buffer Solution (DPBS), Trypsin-ethyleacdiaminetetra-acetic acid (EDTA) solution (0.5ml) was added, and the cells were observed under an inverted microscope to make sure that they were dispersed. Complete growth medium (6.0 to 8.0ml) was added, and the cells were aspirated by gently pipetting. The cell suspension in appropriate aliquots was transferred to new culture plates. The cells were allowed to grow at 37°C in 5% CO₂ for 24 hours before further experiments.

### EXAMPLE 9

### In vitro Cytotoxicity MTT Studies

Polymers conjugates described herein containing doxorubicin are evaluated for their effect on the proliferation of B16F0 melanoma cells at several different concentrations of the drug. Cytotoxic MTT assay is carried out as reported in Monks et al. JNCI 1991, 83, 757-766 . Polymers conjugates are prepared as described in Examples 1-7.

### EXAMPLE 10

### Binding Studies

The binding assays are carried out as described in Line et al, Journal of Nuclear Medicine, 46 (2005), 1552-1560; and Mitra et al., Journal of Controlled Release, 114 (2006) 175-183 . Polymers conjugates described herein are prepared as described in Examples 1-7.

### EXAMPLE 11

### Animals and Tumor Models

Nude mice (6-7 week old, body weight 25-30g, male) are purchased from Charles River Lab (Willington, MA). B16 cell line is purchased from ATCC (CRL-6322, ATCC American Type Culture Collection, Rockville, MD). The B16 cells are cultured in RMPI 1640 supplemented with 10% Fetal bovine serum, 2µM Glutamine, 1mM non-essential amino acids, 1mM sodium pyruvate, 100U/ml penicillin and 100 ug/ml streptomycin. The B16 cells harvested from tissue culture is counted and re-suspended to a concentration of 5 x 10⁶ per mL. Using a TB syringe, 0.2 mL (a total of 1 x 10⁶ cells) is administered via subcutaneous injection into each mouse. One tumor is inoculated per animal at the right hip. The site of tumor inoculation is shaved prior to inoculation to make it easier to measure the tumor as it grows.

### EXAMPLE 12

### Magnetic resonance imaging for tumor accumulation

Images of mice is acquired on a GE 3T MR scanner using a knee coil pre-and post-contrast. The following imaging parameters are TE: minful, TR= 250 ms, FOV: 8 and 24 slices/slab, and 1.0 mm coronal slice thickness. PEG-PGGA-Dox-RGD-[(DTPA)Gd(III)] is prepared as in Example 7. The control material is Omniscan-Gd(III)-(DTPA-BMA (0.1 mmol Gd(III)/ kg). The dose of injection of PEG-PGGA-Dox-RGD-[(DTPA)Gd(III)] and Omniscan™ is 0.1 mmol Gd(III)/kg. The two compounds are injected via a tail vein into anesthetized mice and images are acquired at pre-injection and at 6 minutes to 4 hours post-injection of the contrast agents.

## Claims

1. A copolymer conjugate comprising a recurring unit of the formula (I) and a recurring unit of the formula (II): 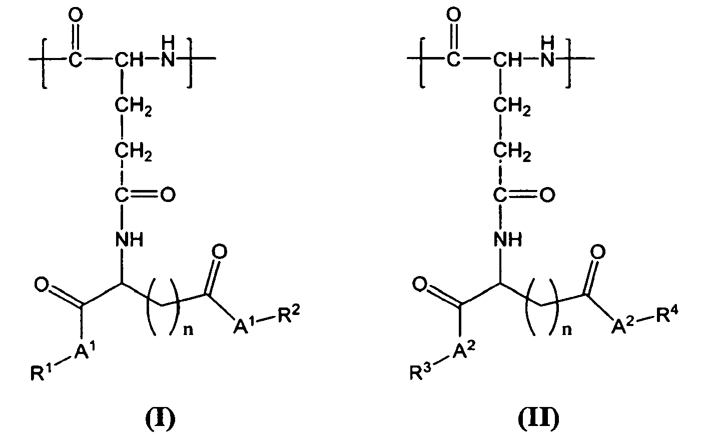 wherein:
each n is independently 1 or 2;
each A¹ and each A² are independently oxygen or NR⁵, wherein each R⁵ is hydrogen or C₁₋₄ alkyl; and
each R¹, each R², each R³ and each R⁴ are independently selected from the group consisting of hydrogen, a C₁₋₁₀ alkyl group, a C₆₋₂₀ aryl group, ammonium, an alkali metal, a polydentate ligand, a polydentate ligand precursor with protected oxygen atoms and a compound that comprises an agent,
wherein the agent is independently selected from the group consisting of an anticancer drug, a targeting agent, an optical imaging agent, a magnetic resonance imaging agent and polyethylene glycol; and
wherein the targeting agent is selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF) and an antibody; or
wherein the targeting agent interacts with a receptor selected from the group consisting of αᵥ,β₃-integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor;
provided that at least one of R¹ and R² is a compound that comprises an anticancer drug; and
at least one of R³ and R⁴ is a polydentate ligand, a polydentate ligand precursor with protected oxygen atoms or a compound that comprises an agent selected from the group consisting of a targeting agent, an optical imaging agent, a magnetic resonance imaging agent and polyethylene glycol.

2. The polymer conjugate of Claim 1, further comprising a recurring unit of the formula (III): 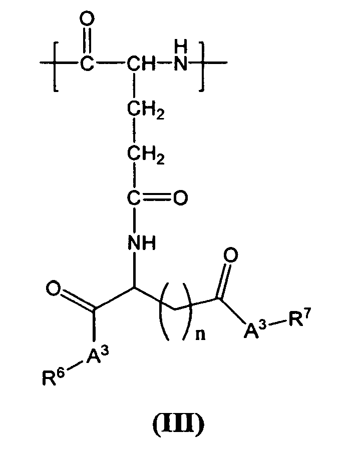 wherein:
each A³ is oxygen; and
each R⁶ and each R⁷ are each independently selected from the group consisting of hydrogen, ammonium and an alkali metal.

3. The polymer conjugate of any one of Claims 1 to 2, further comprising a recurring unit of the formula (IV): 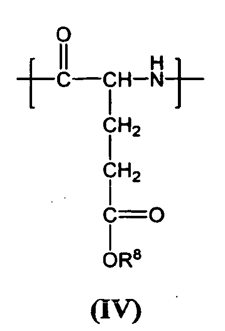 wherein each R⁸ is hydrogen, ammonium or an alkali metal.

4. The polymer conjugate of any one of Claims 1 to 3, wherein the compound that comprises the agent further comprises a linker group.

5. The polymer conjugate of any one of Claims 1 to 4, wherein at least one of R' and R² is a compound that comprises an anticancer drug, and the other of R¹ and R² is selected from the group consisting of hydrogen, ammonium, and an alkali metal; and wherein at least one of R³ and R⁴ is a polydentate ligand, a polydentate ligand precursor with protected oxygen atoms or a compound that comprises an agent selected from the group consisting of a targeting agent, an optical imaging agent, a magnetic resonance imaging agent and polyethylene glycol, and the other of R³ and R⁴ is selected from the group consisting of hydrogen, ammonium, and an alkali metal.

6. The polymer conjugate of any one of Claims 1 to 5, wherein at least one n is 1, or at least one n is 2.

7. The polymer conjugate of any one of Claims 1 to 5, wherein each n is 2.

8. A method of making the polymer conjugate of any one of Claims 1 to 7, comprising the steps of:
dissolving or partially dissolving a polymeric reactant comprising a recurring unit of formula (V) in a solvent to form a dissolved or partially dissolved polymeric reactant; 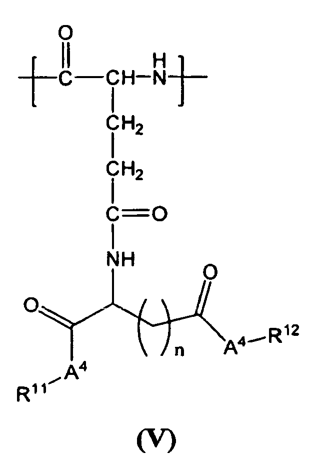 wherein:
each n is independently 1 or 2;
each A⁴ is oxygen; and
each R¹¹ and each R¹² are each independently selected from the group consisting of hydrogen, ammonium and an alkali metal; and
reacting the dissolved or partially dissolved polymeric reactant with a second reactant and a third reactant,
wherein the second reactant comprises the anticancer drug; and
wherein the third reactant comprises the polydentate ligand, the polydentate ligand precursor with protected oxygen atoms or a compound that comprises an agent;
wherein the agent is independently selected from the group consisting of a targeting agent, an optical imaging agent, a magnetic resonance imaging agent and polyethylene glycol; and
wherein the targeting agent is selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF) and an antibody; or
the targeting agent interacts with a receptor selected from the group consisting of αᵥ,β₃-integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor.

9. The method of Claim 8, wherein the second reactant comprises a substituent selected from the group consisting of hydroxy and amine.

10. The method of Claim 8 or 9, wherein the third reactant comprises a substituent selected from the group consisting of hydroxy and amine.

11. The method of any one of Claims 8 to 10, comprising reacting the dissolved or partially dissolved polymer reactant with at least a portion of the second reactant before reacting with the third reactant; or reacting the dissolved or partially dissolved polymer reactant with at least a portion of the second reactant at about the same time as reacting with the third reactant; or reacting the dissolved or partially dissolved polymer reactant with at least a portion of the third reactant before reacting with the second reactant.

12. The polymer conjugate of any one of Claims 1 to 7 or the method of any one of Claims 8 to 11, wherein the polymer conjugate comprises an amount of each agent in the range of about 1 to about 50% (weight/weight) based on the mass ratio of each agent to the polymer conjugate.

13. The polymer conjugate of any of Claims 1 to 7 or the method of any one of Claims 8 to 11, wherein the polymer conjugate comprises an amount of each agent in the range of about 5 to about 40% (weight/weight) based on the mass ratio of each agent to the polymer conjugate.

14. The polymer conjugate of any of Claims 1 to 7, 12, and 13 or the method of any one of Claims 8 to 13, wherein the anticancer drug is selected from the group consisting of a taxane, camptotheca and anthracycline.

15. The polymer conjugate or the method of Claim 14, wherein the anticancer drug is a taxane is selected from the group consisting of paclitaxel and docetaxel.

16. The polymer conjugate or the method of Claim 14, wherein the camptotheca is camptothecin, or wherein the anthracycline is doxorubicin.

17. The polymer conjugate of any of Claims 1 to 7 and 12 to 16 or the method of any one of Claims 8 to 16, wherein one of the agents is an optical imaging agent selected from the group consisting of an acridine dye, a coumarine dye, a rhodamine dye, a xanthene dye, a cyanine dye and a pyrene dye.

18. The polymer conjugate of any of Claims 1 to 7 and 12 to 17 or the method of any one of Claims 8 to 17, wherein one of the agents is a targeting agent selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF) and an antibody.

19. The polymer conjugate of any of Claims 1 to 7 and 12 to 18 or the method of any one of Claims 8 to 18, wherein one of the agents is polyethylene glycol.

20. The polymer conjugate of any of Claims 1 to 7 and 12 to 19 or the method of any one of Claims 8 to 19, wherein one of the agents is a magnetic resonance imaging agent that comprises a Gd(III) compound.

21. The polymer conjugate or method of Claim 20, wherein the GD(III) compound comprises: 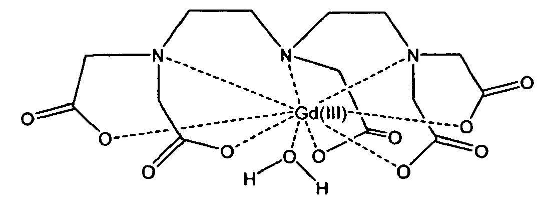 or

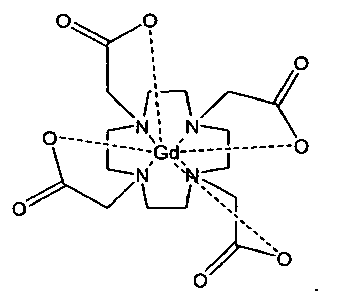

22. The polymer conjugate of any of Claims 1 to 7 and 12 to 21, and the method of any one of Claims 8 to 21, wherein the polydentate ligand comprises: 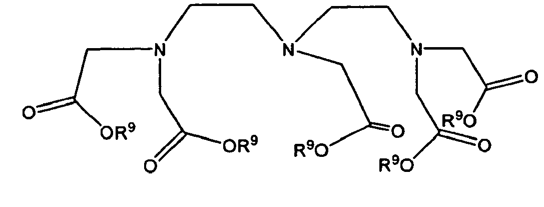 or 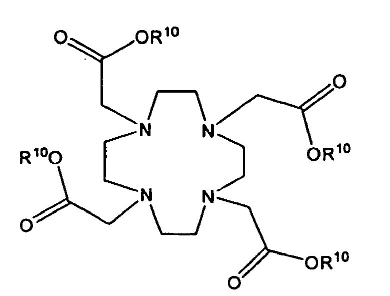 wherein each R⁹ is independently hydrogen, ammonium or an alkali metal; and
each R¹⁰ is independently hydrogen, ammonium or an alkali metal.

23. The polymer conjugate of any of Claims 1 to 7 and 12 to 22, or the method of any one of Claims 8 to 22, wherein the polydentate ligand precursor with protected oxygen atoms comprises:

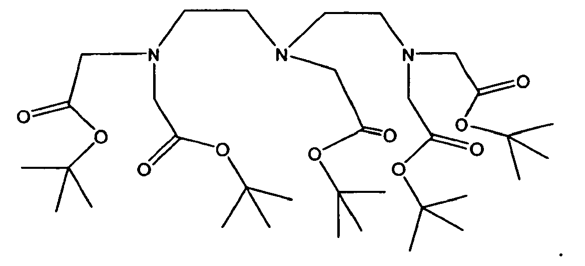

24. A pharmaceutical composition comprising the polymer conjugate of any one of Claims 1 to 7 and 12 to 23, and at least one selected from a pharmaceutically acceptable excipient, a carrier, and a diluent.

25. A polymer conjugate of any one of Claims 1 to 7 and 12 to 23 or a pharmaceutical composition of Claim 24 for use in treating, ameliorating or diagnosing a disease or condition in a mammal.

26. Use of an effective amount of the polymer conjugate of any one of Claims 1 to 7 and 12 to 23 for the preparation of a medicament for treating, ameliorating, or diagnosing a disease or condition in a mammal.

27. The polymer conjugate or pharmaceutical composition of Claim 25 or the use of Claim 26, wherein the disease or condition is selected from the group consisting of lung tumor, breast tumor, colon tumor, ovarian tumor, prostate tumor, melanoma tumor, lung cancer, breast cancer, colon cancer, ovarian cancer, prostate cancer and melanoma.

28. A polymer conjugate of any one of Claims 1 to 7 and 12 to 23, or a pharmaceutical composition of Claim 24 for use in imaging a portion of tissue.

29. Use of an effective amount of the polymer conjugate of any one of Claims 1 to 7 and 12 to 23 for the preparation of a medicament for imaging a portion of tissue.

## Patentansprüche

1. Copolymerkonjugat, das eine sich wiederholende Einheit mit der Formel I und eine sich wiederholende Einheit mit der Formel II umfasst: 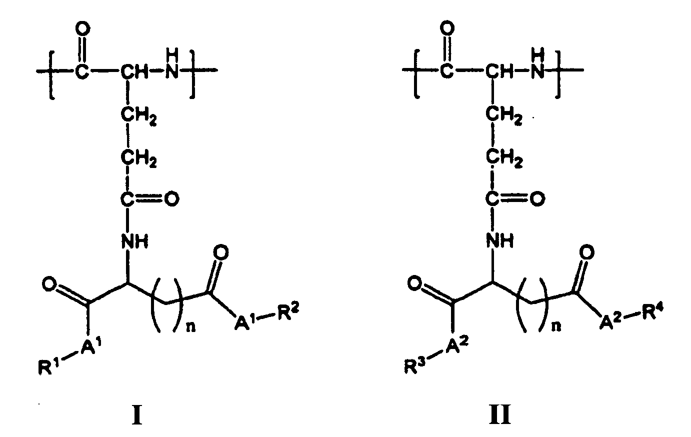 wobei:
jedes n, unabhängig voneinander, 1 oder 2 ist,
jedes A¹ und jedes A², unabhängig voneinander, Sauerstoff oder NR⁵ ist, wobei jedes R⁵ Wasserstoff oder C₁₋₄-Alkyl ist, und
jedes R¹, jedes R², jedes R³ und jedes R⁴, unabhängig voneinander, aus der Gruppe ausgewählt ist, die aus Wasserstoff, einer C₁₋₁₀-Alkyl-Gruppe, einer C₆₋₂₀-Aryl-Gruppe, Ammonium, einem Alkalimetall, einem mehrzähnigen Liganden, einem Vorläufer eines mehrzähnigen Liganden mit geschützten Sauerstoffatomen und einer Verbindung, die ein Agens umfasst, besteht,
wobei das Agens, unabhängig voneinander, aus der Gruppe ausgewählt ist, die aus einem Anti-Krebs-Medikament, einem Targeting-Agens, einem Agens für eine optische Bildgebung, einem Agens für eine Magnetresonanzbildgebung und Polyethylenglykol besteht, und
wobei das Targeting-Agens aus der Gruppe ausgewählt ist, die aus einem Arginin-Glycin-Aspartat (RGD)-Peptid, Fibronectin, Folat, Galactose, einem Apolipoprotein, Insulin, Transferrin, einem Fibroblast Growth Factor (FGF), einem Epidermal Growth Factor (EGF) und einem Antikörper besteht, oder
wobei das Targeting-Agens mit einem Rezeptor interagiert, der aus der Gruppe ausgewählt ist, die aus αᵥ,β₃-Integrin, Folat, Asialoglycoprotein, einem Low-Density-Lipoprotein (LDL), einem Insulinrezeptor, einem Transferrinrezeptor, einem Fibroblast-Growth-Factor (FGF)-Rezeptor, einem Epidermal-Growth-Factor (EGF)-Rezeptor und einem Antikörperrezeptor besteht,
vorausgesetzt, dass wenigstens eines von R¹ und R² eine Verbindung ist, die ein Anti-Krebs-Medikament umfasst, und
wenigstens eines von R³ und R⁴ ein mehrzähniger Ligand, ein Vorläufer eines mehrzähnigen Liganden mit geschützten Sauerstoffatomen oder eine Verbindung ist, die ein Agens umfasst, aus der Gruppe ausgewählt ist, die aus einem Targeting-Agens, einem Agens für eine optische Bildgebung, einem Agens für eine Magnetresonanzbildgebung und Polyethylenglykol besteht.

2. Polymerkonjugat nach Anspruch 1, das ferner eine sich wiederholende Einheit mit der Formel III umfasst: 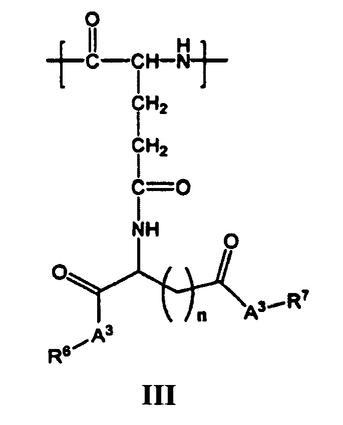 wobei:
jedes A³ Sauerstoff ist und
jedes R⁶ und jedes R⁷ jeweils, unabhängig voneinander, aus der Gruppe ausgewählt ist, die aus Wasserstoff, Ammonium und einem Alkalimetall besteht.

3. Polymerkonjugat nach irgendeinem der Ansprüche 1 bis 2, das ferner eine sich wiederholende Einheit mit der Formel IV umfasst: 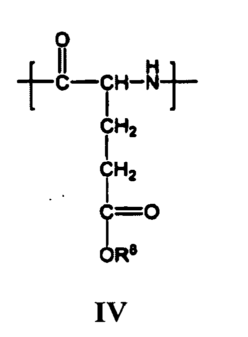 wobei jedes R⁸ Wasserstoff, Ammonium oder ein Alkalimetall ist.

4. Polymerkonjugat nach irgendeinem der Ansprüche 1 bis 3, wobei die Verbindung, die das Agens umfasst, ferner eine Linkergruppe umfasst.

5. Polymerkonjugat nach irgendeinem der Ansprüche 1 bis 4, wobei wenigstens eines von R¹ und R² eine Verbindung ist, die ein Anti-Krebs-Medikament umfasst, und das andere von R¹ und R² aus der Gruppe ausgewählt ist, die aus Wasserstoff, Ammonium und einem Alkalimetall besteht, und wobei wenigstens eines von R³ und R⁴ ein mehrzähniger Ligand, ein Vorläufer eines mehrzähnigen Liganden mit geschützten Sauerstoffatomen oder eine Verbindung ist, die ein Agens umfasst, das aus der Gruppe ausgewählt ist, die aus einem Targeting-Agens, einem Agens für eine optische Bildgebung, einem Agens für eine Magnetresonanzbildgebung und Polyethylenglykol besteht, und das andere von R³ und R⁴ aus der Gruppe ausgewählt ist, die aus Wasserstoff, Ammonium und einem Alkalimetall besteht.

6. Polymerkonjugat nach irgendeinem der Ansprüche 1 bis 5, wobei wenigstens ein n 1 ist oder wenigstens ein n 2 ist.

7. Polymerkonjugat nach irgendeinem der Ansprüche 1 bis 5, wobei jedes n 2 ist.

8. Verfahren zur Herstellung des Polymerkonjugats nach irgendeinem der Ansprüche 1 bis 7, wobei das Verfahren die folgenden Schritte umfasst:
Lösen oder partielles Lösen eines polymeren Reaktionspartners, der eine sich wiederholende Einheit mit der Formel V umfasst, in einem Lösemittel unter Bildung eines gelösten oder partiell gelösten polymeren Reaktionspartners, 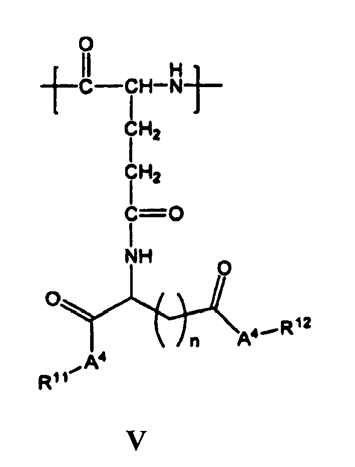 wobei:
jedes n, unabhängig voneinander, 1 oder 2 ist,
jedes A⁴ Sauerstoff ist und
jedes R¹¹ und jedes R¹² jeweils, unabhängig voneinander, aus der Gruppe ausgewählt ist, die aus Wasserstoff, Ammonium und einem Alkalimetall besteht, und
Umsetzen des gelösten oder partiell gelösten polymeren Reaktionspartners mit einem zweiten Reaktionspartner und einem dritten Reaktionspartner,
wobei der zweite Reaktionspartner das Anti-Krebs-Medikament umfasst und
wobei der dritte Reaktionspartner den mehrzähnigen Liganden, den Vorläufer des mehrzähnigen Liganden mit geschützten Sauerstoffatomen oder eine Verbindung umfasst, die ein Agens umfasst,
wobei das Agens, unabhängig voneinander, aus der Gruppe ausgewählt ist, die aus einem Targeting-Agens, einem Agens für eine optische Bildgebung, einem Agens für eine Magnetresonanzbildgebung und Polyethylenglykol besteht, und
wobei das Targeting-Agens aus der Gruppe ausgewählt ist, die aus einem Arginin-Glycin-Aspartat (RGD)-Peptid, Fibronectin, Folat, Galactose, einem Apolipoprotein, Insulin, Transferrin, einem Fibroblast Growth Factor (FGF), einem Epidermal Growth Factor (EGF) und einem Antikörper besteht, oder
das Targeting-Agens mit einem Rezeptor interagiert, der aus der Gruppe ausgewählt ist, die aus αᵥ,β₃-Integrin, Folat, Asialoglycoprotein, einem Low-Density-Lipoprotein (LDL), einem Insulinrezeptor, einem Transferrinrezeptor, einem Fibroblast-Growth-Factor (FGF)-Rezeptor, einem Epidermal-Growth-Factor (EGF)-Rezeptor und einem Antikörperrezeptor besteht.

9. Verfahren nach Anspruch 8, wobei der zweite Reaktionspartner einen Substituenten umfasst, der aus der Gruppe ausgewählt ist, die aus Hydroxy und Amino besteht.

10. Verfahren nach Anspruch 8 oder 9, wobei der dritte Reaktionspartner einen Substituenten umfasst, der aus der Gruppe ausgewählt ist, die aus Hydroxy und Amino besteht.

11. Verfahren nach irgendeinem der Ansprüche 8 bis 10, das umfasst das Umsetzen des gelösten oder partiell gelösten Polymer-Reaktionspartners mit wenigstens einem Teil des zweiten Reaktionspartners vor dem Umsetzen mit dem dritten Reaktionspartner oder das Umsetzen des gelösten oder partiell gelösten Polymer-Reaktionspartners mit wenigstens einem Teil des zweiten Reaktionspartners ungefähr gleichzeitig mit dem Umsetzen mit dem dritten Reaktionspartner oder das Umsetzen des gelösten oder partiell gelösten Polymer-Reaktionspartners mit wenigstens einem Teil des dritten Reaktionspartners vor dem Umsetzen mit dem zweiten Reaktionspartner.

12. Polymerkonjugat nach irgendeinem der Ansprüche 1 bis 7 oder Verfahren nach irgendeinem der Ansprüche 8 bis 11, wobei das Polymerkonjugat eine Menge eines jeden Agens im Bereich von ungefähr 1 bis ungefähr 50 % (Gewicht/Gewicht) basierend auf dem Verhältnis der Masse eines jeden Agens zu derjenigen des Polymerkonjugats umfasst.

13. Polymerkonjugat nach irgendeinem der Ansprüche 1 bis 7 oder Verfahren nach irgendeinem der Ansprüche 8 bis 11, wobei das Polymerkonjugat eine Menge eines jeden Agens im Bereich von ungefähr 5 bis ungefähr 40 % (Gewicht/Gewicht) basierend auf dem Verhältnis der Masse eines jeden Agens zu derjenigen des Polymerkonjugats umfasst.

14. Polymerkonjugat nach irgendeinem der Ansprüche 1 bis 7, 12 und 13 oder Verfahren nach irgendeinem der Ansprüche 8 bis 13, wobei das Anti-Krebs-Medikament aus der Gruppe ausgewählt ist, die aus einem Taxan, einer Camptotheca-Verbindung und Anthracyclin besteht.

15. Polymerkonjugat oder Verfahren nach Anspruch 14, wobei das Anti-Krebs-Medikament ein Taxan ist, das aus der Gruppe ausgewählt ist, die aus Paclitaxel und Docetaxel besteht.

16. Polymerkonjugat oder Verfahren nach Anspruch 14, wobei die Camptotheca-Verbindung Camptothecin ist oder wobei das Anthracyclin Doxorubicin ist.

17. Polymerkonjugat nach irgendeinem der Ansprüche 1 bis 7 und 12 bis 16 oder Verfahren nach irgendeinem der Ansprüche 8 bis 16, wobei eines der Agenzien ein Agens für eine optische Bildgebung ist, das aus der Gruppe ausgewählt ist, die aus einem Acridinfarbstoff, einem Cumarinfarbstoff, einem Rhodaminfarbstoff, einem Xanthenfarbstoff, einem Cyaninfarbstoff und einem Pyrenfarbstoff besteht.

18. Polymerkonjugat nach irgendeinem der Ansprüche 1 bis 7 und 12 bis 17 oder Verfahren nach irgendeinem der Ansprüche 8 bis 17, wobei eines der Agenzien ein Targeting-Agens ist, das aus der Gruppe ausgewählt ist, die aus einem Arginin-Glycin-Aspartat (RGD)-Peptid, Fibronectin, Folat, Galactose, einem Apolipoprotein, Insulin, Transferrin, einem Fibroblast Growth Factor (FGF), einem Epidermal Growth Factor (EGF) und einem Antikörper besteht.

19. Polymerkonjugat nach irgendeinem der Ansprüche 1 bis 7 und 12 bis 18 oder Verfahren nach irgendeinem der Ansprüche 8 bis 18, wobei eines der Agenzien Polyethylenglykol ist.

20. Polymerkonjugat nach irgendeinem der Ansprüche 1 bis 7 und 12 bis 19 oder Verfahren nach irgendeinem der Ansprüche 8 bis 19, wobei eines der Agenzien ein Agens für eine Magnetresonanzbildgebung ist, das eine Gd(III)-Verbindung umfasst.

21. Polymerkonjugat oder Verfahren nach Anspruch 20, wobei die Gd(III)-Verbindung umfasst: 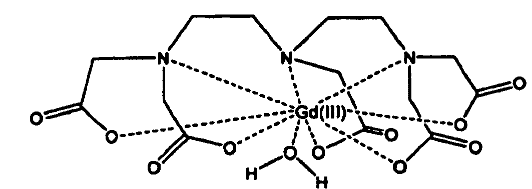 oder

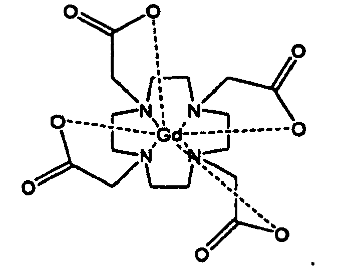

22. Polymerkonjugat nach irgendeinem der Ansprüche 1 bis 7 und 12 bis 21 und Verfahren nach irgendeinem der Ansprüche 8 bis 21, wobei der mehrzähnige Ligand umfasst: 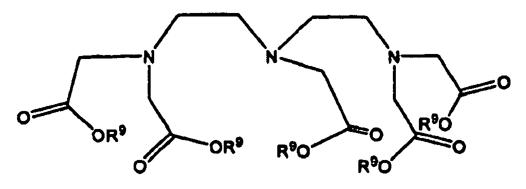 oder 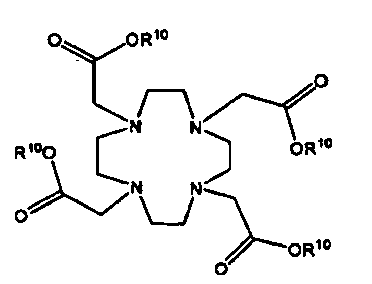 wobei jedes R⁹, unabhängig voneinander, Wasserstoff, Ammonium oder ein Alkalimetall ist und jedes R¹⁰, unabhängig voneinander, Wasserstoff, Ammonium oder ein Alkalimetall ist.

23. Polymerkonjugat nach irgendeinem der Ansprüche 1 bis 7 und 12 bis 22 oder Verfahren nach irgendeinem der Ansprüche 8 bis 22, wobei der Vorläufer des mehrzähnigen Liganden mit geschützten Sauerstoffatomen umfasst:

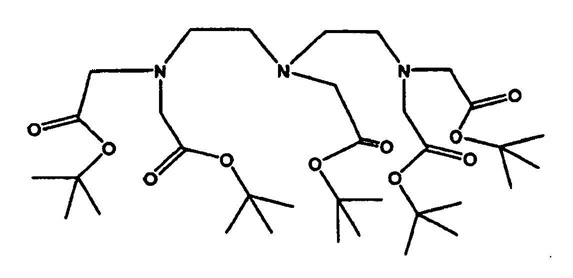

24. Pharmazeutische Zusammensetzung, die das Polymerkonjugat nach irgendeinem der Ansprüche 1 bis 7 und 12 bis 23 und wenigstens eine aus einem pharmazeutisch annehmbaren Hilfsstoff, einem Träger und einem Verdünnungsmittel ausgewählte Komponente umfasst.

25. Polymerkonjugat nach irgendeinem der Ansprüche 1 bis 7 und 12 bis 23 oder pharmazeutische Zusammensetzung nach Anspruch 24 für die Verwendung bei der Behandlung, Linderung oder Diagnose einer Krankheit oder eines Leidens eines Säugetiers.

26. Verwendung einer wirksamen Menge des Polymerkonjugats nach irgendeinem der Ansprüche 1 bis 7 und 12 bis 23 zur Zubereitung eines Medikaments für die Behandlung, Linderung oder Diagnose einer Krankheit oder eines Leidens eines Säugetiers.

27. Polymerkonjugat oder pharmazeutische Zusammensetzung nach Anspruch 25 oder Verwendung nach Anspruch 26, wobei die Krankheit oder das Leiden aus der Gruppe ausgewählt ist, die aus einem Lungentumor, Brusttumor, Kolontumor, Ovarialtumor, Prostatatumor, Melanomtumor, Lungenkrebs, Brustkrebs, Kolonkrebs, Ovarialkrebs, Prostatakrebs und Melanom besteht.

28. Polymerkonjugat nach irgendeinem der Ansprüche 1 bis 7 und 12 bis 23 oder pharmazeutische Zusammensetzung nach Anspruch 24 für die Verwendung zur bildgebenden Darstellung eines Teils eines Gewebes.

29. Verwendung einer wirksamen Menge des Polymerkonjugats nach irgendeinem der Ansprüche 1 bis 7 und 12 bis 23 zur Zubereitung eines Medikaments für die Bildgebung eines Teils eines Gewebes.

## Revendications

1. Conjugué de copolymère comprenant un motif récurrent de formule (I) et un motif récurrent de formule (II) _{:} 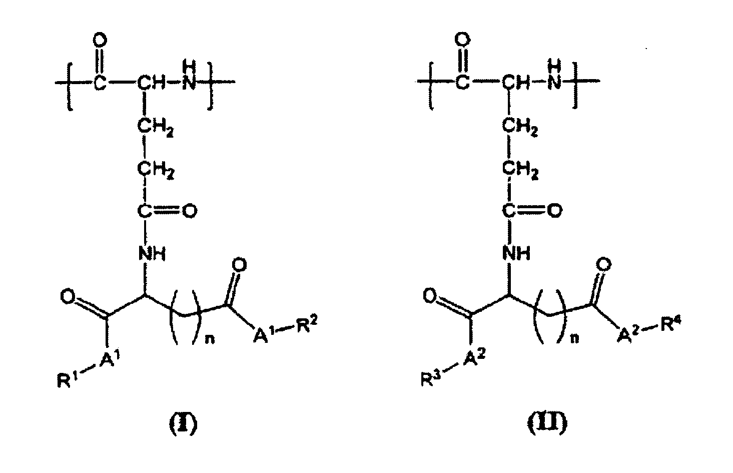 dans lequel :
chaque n vaut indépendamment 1 ou 2 ;
chaque A¹ et chaque A² sont indépendamment un oxygène ou NR⁵, où chaque R⁵ est un hydrogène ou un alkyle en C₁₋₄ ; et
chaque R¹, chaque R², chaque R³ et chaque R⁴ sont indépendamment choisis dans le groupe consistant en un hydrogène, un groupe alkyle en C₁₋₁₀, un groupe aryle en C₆₋₂₀, un ammonium, un métal alcalin, un ligand polydentate, un précurseur de ligand polydentate avec des atomes d'oxygène protégés et un composé qui comprend un agent,
dans lequel l'agent est indépendamment choisi dans le groupe consistant en un médicament anticancéreux, un agent de ciblage, un agent d'imagerie optique, un agent d'imagerie par résonance magnétique et du poly(éthylène glycol) ; et
dans lequel l'agent de ciblage est choisi dans le groupe consistant en un peptide arginine-glycine-aspartate (RGD), la fibronectine, le folate, le galactose, une apolipoprotéine, l'insuline, la transferrine, un facteur de croissance des fibroblastes (FGF), un facteur de croissance épidermique (EGF) et un anticorps ; ou
dans lequel l'agent de ciblage interagit avec un récepteur choisi dans le groupe consistant en l'αᵥ,β₃-intégrine, le folate, l'asialoglycoprotéine, une lipoprotéine basse densité (LDL), un récepteur de l'insuline, un récepteur de la transferrine, un récepteur du facteur de croissance des fibroblastes (FGF), un récepteur du facteur de croissance épidermique (EGF) et un récepteur d'anticorps ;
à condition qu'au moins l'un de R¹ et R² soit un composé qui comprend un médicament anticancéreux ; et
au moins l'un de R³ et R⁴ est un ligand polydentate, un précurseur de ligand polydentate avec des atomes d'oxygène protégés ou un composé qui comprend un agent choisi dans le groupe consistant en un agent de ciblage, un agent d'imagerie optique, un agent d'imagerie par résonance magnétique et du poly(éthylène glycol).

2. Conjugué de polymère selon la revendication 1, comprenant en outre un motif récurrent de formule (III) : 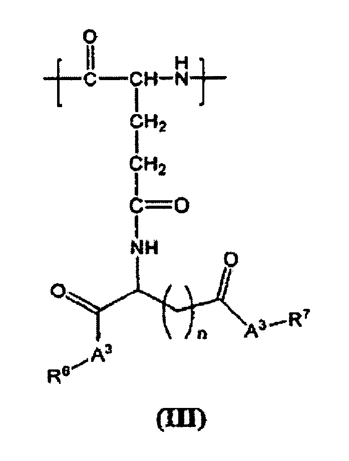 dans lequel :
chaque A³ est un oxygène ; et
chaque R⁶ et chaque R⁷ sont chacun indépendamment choisis dans le groupe consistant en un hydrogène, un ammonium et un métal alcalin.

3. Conjugué de polymère selon l'une quelconque des revendications 1 et 2, comprenant en outre un motif récurrent de formule (IV) _{:} 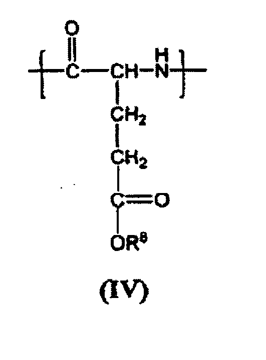 dans lequel chaque R⁸ est un hydrogène, un ammonium ou un métal alcalin.

4. Conjugué de polymère selon l'une quelconque des revendications 1 à 3, dans lequel le composé qui comprend l'agent comprend en outre un groupe lieur.

5. Conjugué de polymère selon l'une quelconque des revendications 1 à 4, dans lequel au moins l'un de R¹ et R² est un composé qui comprend un médicament anticancéreux, et l'autre de R¹ et R² est choisi dans le groupe consistant en un hydrogène, un ammonium et un métal alcalin ; et dans lequel au moins l'un de R³ et R⁴ est un ligand polydentate, un précurseur de ligand polydentate avec des atomes d'oxygène protégés ou un composé qui comprend un agent choisi dans le groupe consistant en un agent de ciblage, un agent d'imagerie optique, un agent d'imagerie par résonance magnétique et du poly(éthylène glycol), et l'autre de R³ et R⁴ est choisi dans le groupe consistant en un atome d'hydrogène, un ammonium et un métal alcalin.

6. Conjugué de polymère selon l'une quelconque des revendications 1 à 5, dans lequel au moins un n vaut 1, ou au moins un n vaut 2.

7. Conjugué de polymère selon l'une quelconque des revendications 1 à 5, dans lequel chaque n vaut 2.

8. Procédé de préparation du conjugué de polymère selon l'une quelconque des revendications 1 à 7, comprenant les étapes de :
dissolution ou dissolution partielle d'un réactif polymérique comprenant un motif récurrent de formule (V) dans un solvant pour former un réactif polymérique dissous ou partiellement dissous ; 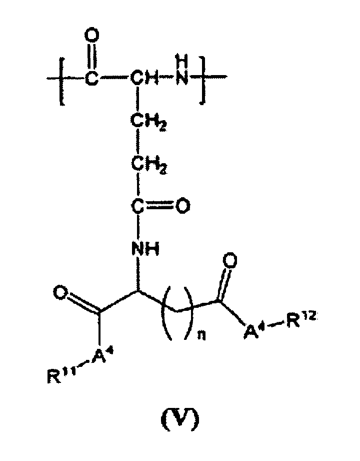
dans lequel :
chaque n vaut indépendamment 1 ou 2 ;
chaque A⁴ est un atome d'oxygène ; et
chaque R¹¹ et chaque R¹² sont chacun indépendamment choisis dans le groupe consistant en un hydrogène, un ammonium et un métal alcalin ; et
réaction du réactif polymérique dissous ou partiellement dissous avec un deuxième réactif et un troisième réactif,
dans lequel le deuxième réactif comprend le médicament anticancéreux ; et
dans lequel le troisième réactif comprend le ligand polydentate, le précurseur de ligand polydentate avec des atomes d'oxygène protégés ou un composé qui comprend un agent ;
dans lequel l'agent est indépendamment choisi dans le groupe consistant en un agent de ciblage, un agent d'imagerie optique, un agent d'imagerie par résonance magnétique et du poly(éthylène glycol) ; et
dans lequel l'agent de ciblage est choisi dans le groupe consistant en un peptide arginine-glycine-aspartate (RGD), la fibronectine, le folate, le galactose, une apolipoprotéine, l'insuline, la transferrine, un facteur de croissance des fibroblastes (FGF), un facteur de croissance épidermique (EGF) et un anticorps ; ou
l'agent de ciblage interagit avec un récepteur choisi dans le groupe consistant en l'αᵥ,β₃-intégrine, le folate, l'asialoglycoprotéine, une lipoprotéine basse densité (LDL), un récepteur de l'insuline, un récepteur de la transferrine, un récepteur du facteur de croissance des fibroblastes (FGF), un récepteur du facteur de croissance épidermique (EGF) et un récepteur d'anticorps.

9. Procédé selon la revendication 8, dans lequel le deuxième réactif comprend un substituant choisi dans le groupe consistant en un hydroxy et une amine.

10. Procédé selon la revendication 8 ou 9, dans lequel le troisième réactif comprend un substituant choisi dans le groupe consistant en un hydroxy et une amine.

11. Procédé selon l'une quelconque des revendications 8 à 10, comprenant la réaction du réactif polymérique dissous ou partiellement dissous avec au moins une portion du deuxième réactif avant la réaction avec le troisième réactif ; ou la réaction du réactif polymérique dissous ou partiellement dissous avec au moins une portion du deuxième réactif à peu près en même temps que la réaction avec le troisième réactif ; ou la réaction du réactif polymérique dissous ou partiellement dissous avec au moins une portion du troisième réactif avant la réaction avec le deuxième réactif.

12. Conjugué de polymère selon l'une quelconque des revendications 1 à 7, ou procédé selon l'une quelconque des revendications 8 à 11, dans lequel le conjugué de polymère comprend une quantité de chaque agent dans la plage d'environ 1 à environ 50 % (poids/poids) par rapport au ratio massique entre chaque agent et le conjugué de polymère.

13. Conjugué de polymère selon l'une quelconque des revendications 1 à 7, ou procédé selon l'une quelconque des revendications 8 à 11, dans lequel le conjugué de polymère comprend une quantité de chaque agent dans la plage d'environ 5 à environ 40 % (poids/poids) par rapport au ratio massique entre chaque agent et le conjugué de polymère.

14. Conjugué de polymère selon l'une quelconque des revendications 1 à 7, 12 et 13, ou procédé selon l'une quelconque des revendications 8 à 13, dans lequel le médicament anticancéreux est choisi dans le groupe consistant en un taxane, un camptothéca et une anthracycline.

15. Conjugué de polymère ou procédé selon la revendication 14, dans lequel le médicament anticancéreux est un taxane choisi dans le groupe consistant en le paclitaxel et le docétaxel.

16. Conjugué de polymère ou procédé selon la revendication 14, dans lequel le camptothéca est la camptothécine, ou dans lequel l'anthracycline est la doxorubicine.

17. Conjugué de polymère selon l'une quelconque des revendications 1 à 7 et 12 à 16, ou procédé selon l'une quelconque des revendications 8 à 16, dans lequel l'un des agents est un agent d'imagerie optique choisi dans le groupe consistant en un colorant acridine, un colorant coumarine, un colorant rhodamine, un colorant xanthène, un colorant cyanine et un colorant pyrène.

18. Conjugué de polymère selon l'une quelconque des revendications 1 à 7 et 12 à 17, ou procédé selon l'une quelconque des revendications 8 à 17, dans lequel l'un des agents est un agent de ciblage choisi dans le groupe consistant en un peptide arginine-glycine-aspartate (RGD), la fibronectine, le folate, le galactose, une apolipoprotéine, l'insuline, la transferrine, un facteur de croissance des fibroblastes (FGF), un facteur de croissance épidermique (EGF) et un anticorps.

19. Conjugué de polymère selon l'une quelconque des revendications 1 à 7 et 12 à 18, ou procédé selon l'une quelconque des revendications 8 à 18, dans lequel l'un des agents est le poly(éthylène glycol).

20. Conjugué de polymère selon l'une quelconque des revendications 1 à 7 et 12 à 19, ou procédé selon l'une quelconque des revendications 8 à 19, dans lequel l'un des agents est un agent d'imagerie par résonance magnétique qui comprend un composé Gd(III).

21. Conjugué de polymère ou procédé selon la revendication 20, dans lequel le composé Gd(III) comprend : 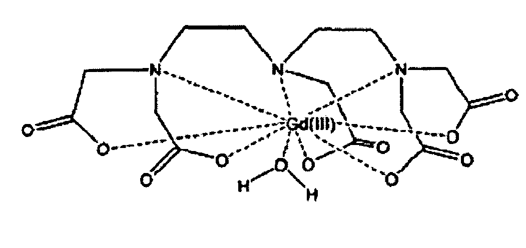 ou

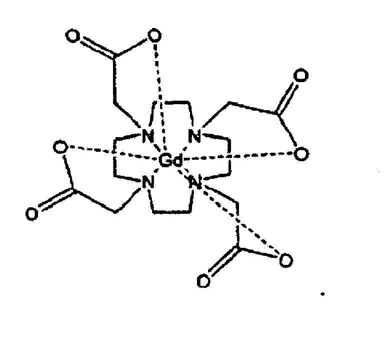

22. Conjugué de polymère selon l'une quelconque des revendications 1 à 7 et 12 à 21, et procédé selon l'une quelconque des revendications 8 à 21, dans lesquels le ligand polydentate comprend : 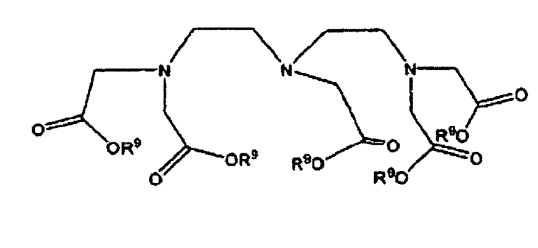 ou 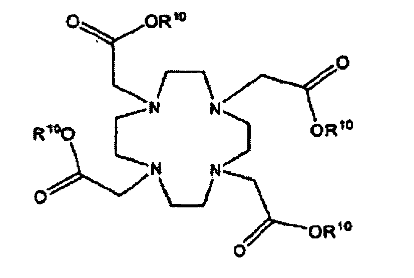 où chaque R⁹ est indépendamment un hydrogène, un ammonium ou un métal alcalin ; et
chaque R¹⁰ est indépendamment un hydrogène, un ammonium ou un métal alcalin.

23. Conjugué de polymère selon l'une quelconque des revendications 1 à 7 et 12 à 22, ou procédé selon l'une quelconque des revendications 8 à 22, dans lequel le précurseur de ligand polydentate avec des atomes d'oxygène protégés comprend :

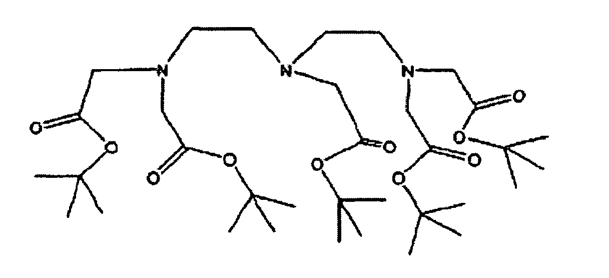

24. Composition pharmaceutique comprenant le conjugué de polymère de l'une quelconque des revendications 1 à 7 et 12 à 23, et au moins un élément choisi parmi un excipient, un vecteur et un diluant pharmaceutiquement acceptables.

25. Conjugué de polymère selon l'une quelconque des revendications 1 à 7 et 12 à 23, ou composition pharmaceutique selon la revendication 24, à utiliser dans le traitement, l'amélioration ou le diagnostic d'une maladie ou d'une affection chez un mammifère.

26. Utilisation d'une quantité efficace du conjugué de polymère de l'une quelconque des revendications 1 à 7 et 12 à 23, pour la préparation d'un médicament destiné à traiter, améliorer ou diagnostiquer une maladie ou une affection chez un mammifère.

27. Conjugué de polymère ou composition pharmaceutique selon la revendication 25 ou utilisation selon la revendication 26, dans lequel ou laquelle la maladie ou l'affection est choisie dans le groupe consistant en une tumeur du poumon, une tumeur du sein, une tumeur du côlon, une tumeur de l'ovaire, une tumeur de la prostate, une tumeur de mélanome, un cancer du poumon, un cancer du sein, un cancer du côlon, un cancer de l'ovaire, un cancer de la prostate et un mélanome.

28. Conjugué de polymère selon l'une quelconque des revendications 1 à 7 et 12 à 23, ou composition pharmaceutique selon la revendication 24, pour une utilisation dans l'imagerie d'une portion de tissu.

29. Utilisation d'une quantité efficace du conjugué de polymère de l'une quelconque des revendications 1 à 7 et 12 à 23, pour la préparation d'un médicament destiné à l'imagerie d'une portion de tissu.
